# EUROPEAN PATENT SPECIFICATION

(11) **EP 4 203 979 B1**
(45) Date of publication and mention of the grant of the patent: **06.12.2023**
(21) Application number: 21778144.2
(22) Date of filing: 22.09.2021
(51) Int. Cl.: A61K 35/15, A61K 39/00, A61P 29/00, A61P 35/00, A61P 37/04, A61P 37/06, C07K 16/28, C12N 5/0784

(54) **TREATING CANCER AND AUTOIMMUNE AND INFLAMMATORY DISEASES**
BEHANDLUNG VON KREBS UND AUTOIMMUN- UND ENTZÜNDUNGSKRANKHEITEN
TRAITEMENT DU CANCER ET DE MALADIES AUTO-IMMUNES ET INFLAMMATOIRES

(30) Priority: 22.09.2020 GB 202014920
(43) Date of publication of application: 05.07.2023
(73) Proprietor: UNIKUM Therapeutics ApS, N 2200 København (DK)
(72) Inventor: BAK, Rasmus Otkjær, 8220 Brabrand (DK); JAKOBSEN, Martin Roelsgaard, 8240 Risskov (DK); LAUSTSEN, Anders, 8210 Aarhus V (DK); NIELSEN, Ulrik, Quincy, Massachusetts 02171 (US)
(74) Representative: J A Kemp LLP
(86) International application number: PCT/EP2021/076051
(87) International publication number: WO 2022/063818

(56) References cited:
- EP-A1- 3 578 642
- WO-A1-2012/158556
- WO-A1-2016/004875
- WO-A1-2018/206577
- US-A1- 2008 267 994
- SABADO RACHEL L ET AL: "Dendritic cell-based immunotherapy", CELL RESEARCH , vol. 27, no. 1 1 January 2017 (2017-01-01), pages 74-95, XP055805715, Singapore ISSN: 1001-0602, DOI: 10.1038/cr.2016.157 Retrieved from the Internet: URL:https://www.nature.com/articles/cr2016 157.pdf

## Description

### Field of the Invention

The present invention relates to engineered cells for use in methods for treating disease, in particular cancer and autoimmune and inflammatory diseases.

### Background of the Invention

Chimeric antigen receptor T-cell (CAR-T) therapy is an established and successful approach to treating cancer. In CAR-T therapy, a T-cell population is obtained from a patient or donor and is engineered to express a chimeric antigen receptor (CAR). The extracellular domain of a typical CAR consists of the VH and VL domains - single-chain fragment variable (scFv) - from the antigen binding sites of a monoclonal antibody that recognises a tumour associated antigen. The scFv is linked to a flexible transmembrane domain followed by an intracellular signalling domain with, for example, a tyrosine-based activation motif such as that from CD3z, and optionally additional activation domains from co-stimulatory molecules such as CD28 and CD137 (41BB), which serve to activate the T-cells and enhance their survival and proliferation. CAR T-cells are administered to the patient and the CAR recognises and binds tumour cells. Binding of cancer cells leads to activation of cytolytic mechanisms in the T-cells, which specifically kill the bound cancer cells. Various preclinical and early-phase clinical trials highlight the efficacy of CAR T cells to treat cancer patients with solid tumours and hematopoietic malignancies.

Obstacles to successful CAR-T therapy include loss of the targeted antigen from tumours, immunosuppressive cells such as Tregs and myeloid-derived suppressor cells (MDSCs) in the tumour microenvironment, factors in the tumour microenvironment such as adenosine, extracellular potassium, and reactive oxygen species (ROS) that can inhibit the cytolytic activity of T-cells, and tumours that express chemokines for which CAR-T cells do not have receptors.

Other cells have been proposed for use with CARs. Natural killer cells are a type of cytotoxic lymphocyte that naturally attack virus-infected cells and tumour cells and can be engineered with CARs to form CAR-NK cells. WO2017019848 describes use of other phagocytic cells capable of cellular cytotoxicity, in particular macrophages.

Plasmacytoid dendritic cells (pDCs) are a rare type of immune cell that are considered to be key in linking the innate and adaptive immune systems. They are known to secrete large quantities of type 1 interferon (IFNs) in response to a viral infection and are key effectors in cellular immunity with the ability to not only initiate immune responses but also to induce tolerance to exogenous and endogenous antigens. Plasmacytoid dendritic cells have been proposed for use in vaccines, where they present antigens and are delivered to lymph nodes to activate T-cells (Charles et al., 2020, Oncology, 9(1)). WO2018/206577 provides methods for generating populations of pDCs.

There remains a need in the art for improved methods for treating cancer and other diseases.

### Summary of the Invention

The inventors have developed an immunotherapy that utilises the unique properties of plasmacytoid dendritic cells (pDCs) to target specific cells, enhance or alter immunological pathways, and treat disease. According to the invention, pDCs are engineered to express on their surface an exogenous construct comprising an extracellular antigen recognition domain that recognises an antigen on a target cell. The extracellular antigen recognition domain allows the pDCs to specifically target disease cells and tissue to activate immune responses and treat disease in the disease microenvironment. The multifaceted nature of pDCs means that they can be used to activate varied effects on immune responses by secreting proinflammatory cytokines or inhibitory factors, or recruiting and activating immune cells such as Tregs or cytotoxic lymphocytes. As a consequence pDCs are expected to exert powerful and pervasive effects, even in disease microenvironments that are refractive to the desired immune response. pDCs may also be capable of antigen presentation to immune cells, and may conduct TRAIL-mediated direct killing of target cells.

In certain embodiments, the exogenous construct also comprises a transmembrane domain and an intracellular signalling domain that activates an immune response in the cell when the extracellular antigen recognition domain binds the target cell. Such constructs allow tailored immune responses to be activated upon binding of the recognition domain to the target antigen.

The Examples demonstrate that pDCs can express exogenous constructs comprising an extracellular antigen recognition domain and other domains. The pDCs maintain their functionality and type I IFN response and can be used to successfully recognise target cells, bind and subsequently activate an immune response.

Accordingly, the invention provides an engineered plasmacytoid dendritic cell for use in a method of treating a disease in a subject, wherein the method comprises administering the engineered cell to the subject, wherein the cell expresses on its surface an exogenous construct comprising an extracellular antigen recognition domain that recognises an antigen on a target cell, and wherein the cell activates an immune response when the extracellular antigen recognition domain binds the target cell.

The invention also provides an engineered plasmacytoid dendritic cell that expresses on its surface an exogenous construct comprising an extracellular antigen recognition domain that recognises an antigen on a target cell.

In preferred embodiments, the exogenous construct further comprises a transmembrane domain and an intracellular signalling domain that activates an immune response in the cell when the extracellular antigen recognition domain binds the target cell.

In preferred embodiments, the cells of the invention are for use in treating cancer or an autoimmune or inflammatory disease. The ability of pDCs to secrete immunomodulatory factors and alter immune-inhibiting or inflammatory environments are expected to be particularly useful for treating such diseases, where disease-causing cells reside in and are maintained by particular immune microenvironments. Also, cancer cells, autoreactive T-cells and auto-antigens present specific antigens that can be recognised by the extracellular antigen recognition domain to provide targeted therapy. Treatment of inflammatory diseases and transplant rejection will also benefit from pDCs that are able to specifically target disease tissue.

In preferred embodiments, the construct is a chimeric antigen receptor (CAR) or a synthetic Notch receptor. Such constructs can effectively target specific cells using their extracellular antigen recognition domains and can provide tailored immune responses with their intracellular signalling domains.

In preferred embodiments, the immune response activated comprises secretion of cytokines, such as pro-inflammatory or anti-inflammatory cytokines.

In further preferred embodiments, the immune response comprises recruitment or activation of host immune cells, such as Tregs or cytotoxic lymphocytes. This may include antigen presentation to host immune cells by the pDC.

The pDC may also perform direct TRAIL-mediated killing of target cells.

In preferred embodiments, the pDC is a stem cell-derived plasmacytoid dendritic cell (SC-pDC). Such cells can be generated from hematopoietic stem and progenitor cells (HSPCs), which can be obtained from a patient or a donor. The HSPCs may be engineered with the exogenous construct before differentiation to SC-pDCs.

In especially preferred embodiments, the invention provides an engineered cell for use in a method of treating cancer in a subject, wherein the method comprises administering the engineered cell to the subject, wherein the cell expresses on its surface an exogenous chimeric antigen receptor comprising an extracellular antigen recognition domain that recognises an antigen on a target cell, a transmembrane domain, and an intracellular signalling domain that activates an immune response in the cell when the extracellular antigen recognition domain binds the target cell, wherein the cell is a stem cell-derived plasmacytoid dendritic cell.

In further especially preferred embodiments, the invention provides an engineered cell for use in a method of treating an autoimmune disease in a subject, wherein the method comprises administering the engineered cell to the subject, wherein the cell expresses on its surface an exogenous chimeric antigen receptor comprising an extracellular antigen recognition domain that recognises an antigen on a target cell, a transmembrane domain, and an intracellular signalling domain that activates an immune response in the cell when the extracellular antigen recognition domain binds the target cell, wherein the cell is a stem cell-derived plasmacytoid dendritic cell.

Similarly, in especially preferred embodiments, the invention provides an engineered stem cell-derived plasmacytoid dendritic cell that expresses an exogenous chimeric antigen receptor comprising an extracellular antigen recognition domain that recognises an antigen on a target cell, a transmembrane domain, and an intracellular signalling domain that activates an immune response in the cell when the extracellular antigen recognition domain binds the target cell.

In further especially preferred embodiments, the invention provides an engineered cell for use in a method of treating cancer in a subject, wherein the method comprises administering the engineered cell to the subject, wherein the cell expresses an exogenous synthetic Notch receptor comprising an extracellular antigen recognition domain that recognises an antigen on a target cell, a transmembrane domain, and an intracellular signalling domain, such as a transcription factor, that activates an immune response in the cell when the extracellular antigen recognition domain binds the target cell, wherein the cell is a stem cell-derived plasmacytoid dendritic cell.

In further especially preferred embodiments, the invention provides an engineered cell for use in a method of treating an autoimmune disease in a subject, wherein the method comprises administering the engineered cell to the subject, wherein the cell expresses an exogenous synthetic Notch receptor comprising an extracellular antigen recognition domain that recognises an antigen on a target cell, a transmembrane domain, and an intracellular signalling domain that activates an immune response in the cell when the extracellular antigen recognition domain binds the target cell, wherein the cell is a stem cell-derived plasmacytoid dendritic cell.

Similarly, in especially preferred embodiments, the invention provides an engineered stem cell-derived plasmacytoid dendritic cell that expresses an exogenous synthetic Notch receptor comprising an extracellular antigen recognition domain that recognises an antigen on a target cell, a transmembrane domain, and an intracellular signalling domain that activates an immune response in the cell when the extracellular antigen recognition domain binds the target cell.

The invention also provides a pharmaceutical composition comprising an effective amount of an engineered plasmacytoid dendritic cell described herein and a pharmaceutically acceptable excipient.

The invention also provides the engineered plasmacytoid dendritic cells of the invention for use in treating disease, such as for use in treating cancer or an inflammatory or autoimmune disease.

The invention also provides use of the engineered plasmacytoid dendritic cells of the invention in the preparation of a medicament for treating disease, such as a medicament for treating cancer or an inflammatory or autoimmune disease.

In further embodiments, the invention provides a method for producing an engineered plasmacytoid dendritic cell (pDC) comprising:
- providing hematopoietic stem progenitor cells (HSPCs)
- transforming said HSPCs with an exogenous construct comprising an extracellular antigen recognition domain that recognises an antigen on a target cell,
- incubating said HSPCs in one or more media, which media may typically comprise one or more cytokines, growth factors, interferons (IFNs) and/or aryl hydrocarbon receptor (AHR) antagonists (such as stemregenin-1), whereby said HSPCs are differentiated into precursor-pDCs and into pDCs.

In certain embodiments, the invention provides a method for producing an engineered plasmacytoid dendritic cell (pDC) comprising:
- providing hematopoietic stem progenitor cells (HSPCs),
- incubating said HSPCs in one or more media, which media may typically comprise one or more cytokines, growth factors, interferons (IFNs) and/or aryl hydrocarbon receptor (AHR) antagonists (such as stemregenin-1), whereby said HSPCs are differentiated into precursor-pDCs and into pDCs.
- transforming said pDCs with an exogenous construct comprising an extracellular antigen recognition domain that recognises an antigen on a target cell.

### Brief Description of the Figures

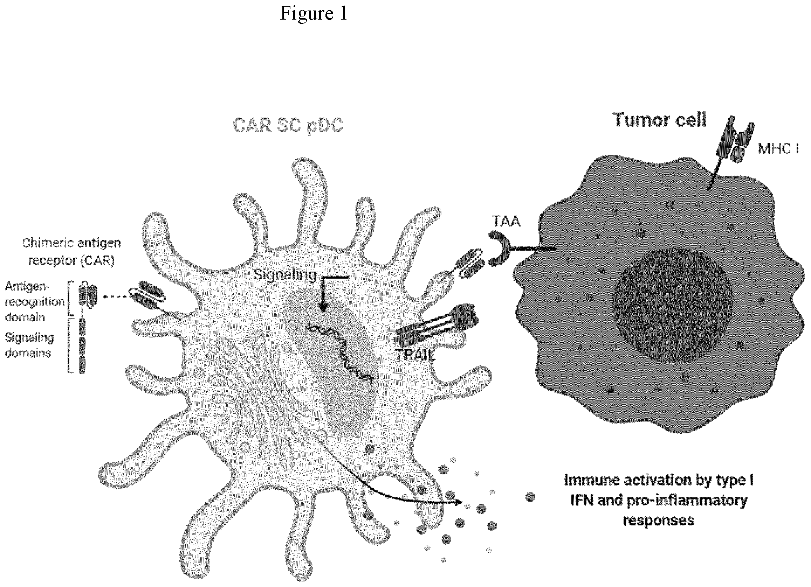

Figure 1 - **SC-pDC expressing a chimeric antigen receptor (CAR) recognising a tumour cell.** The CAR recognizes a specific tumour-associated antigen (TAA) through an extracellular antigen recognition domain. Binding of the CAR SC-pDCs to the TAA leads to activation of the pDC through a signalling domain in the CAR, resulting in secretion of proinflammatory cytokines and interferon by the pDC, promoting anti-tumour responses.

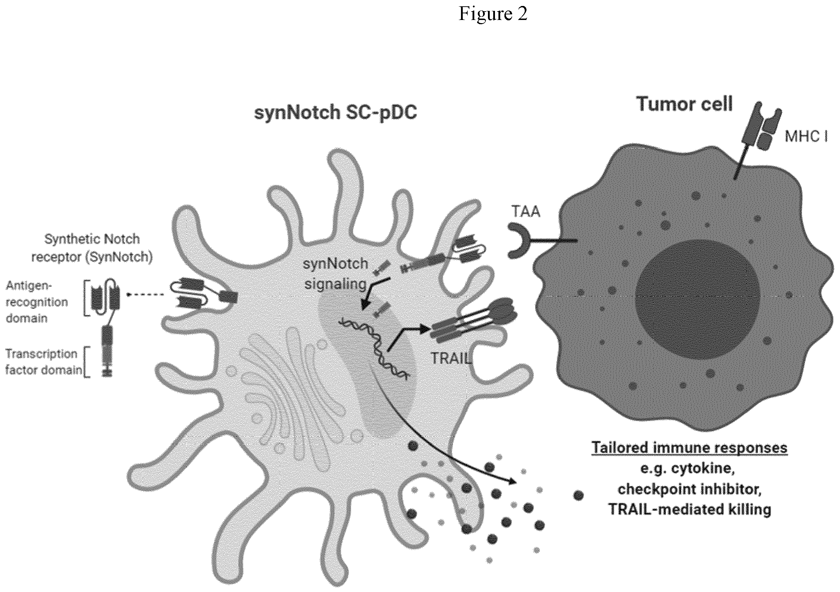

Figure 2 - **SC-pDC expressing a synthetic Notch (synNotch) receptor recognising a tumour cell.** Upon receptor binding to a specific molecule, e.g. a tumour associated molecule, a specific tailored synthetic immune response is induced in the SC-pDCs, allowing for directly tailored responses.

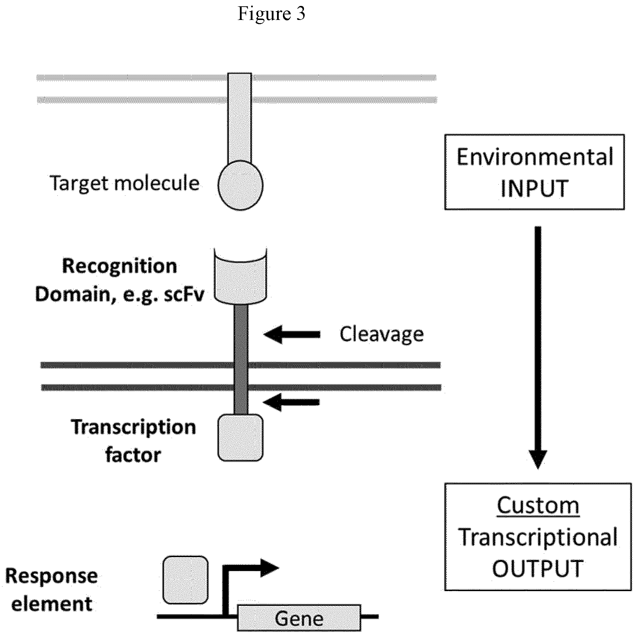

Figure 3 - **Structure of a synNotch receptor.** The synNotch receptor comprises a custom extracellular recognition domain (e.g. scFv), a core regulatory region (controls proteolysis/cleavage) and a custom cytoplasmic transcription factor (e.g. Gal4-VP64). The custom response element with recognition elements for the transcription factor activates expression of a custom gene. The system provide control of the specific response activated in the cell by directly regulating a custom transcriptional program.

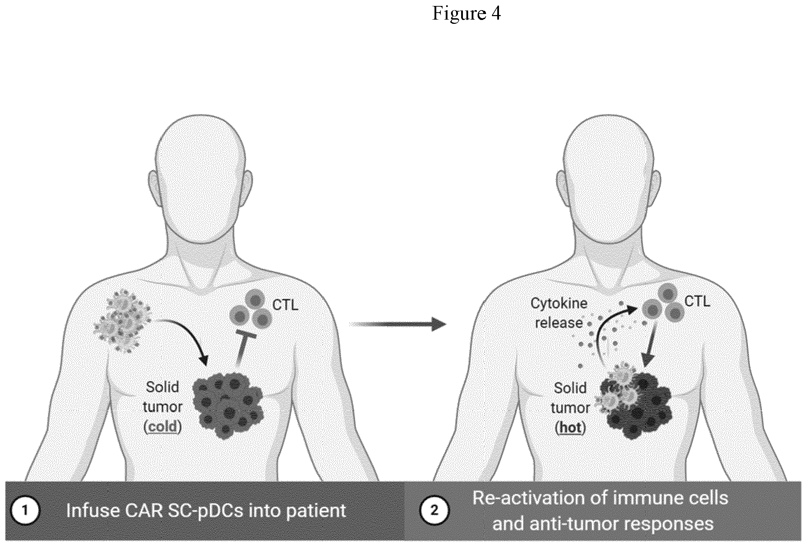

Figure 4 - **Infusion of CAR SC-pDC to a cancer patient.** SC-pDCs expressing a CAR are injected into a patient. The CAR SC-pDCs will home to the tumour site and upon recognition of the specific tumour antigen will become activated and secrete pro-inflammatory cytokines and interferon. This will in turn promote trafficking of cytotoxic T lymphocytes (CTL) to the tumour site. Moreover, the pro-inflammatory environment will abolish the immune-inhibiting environment that tumour cells generate, transforming a "cold" tumour into a "hot" tumour and promoting anti-tumour responses.

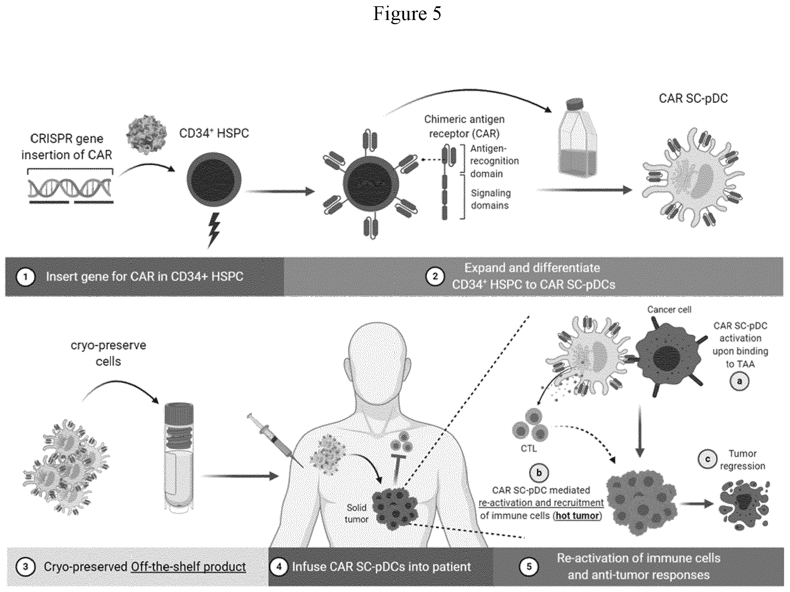

Figure 5 - **Exemplary process for generating and administrating CAR SC-pDCs for targeted cancer therapy.** The CAR gene can be inserted into CD34+ HSPCs using either lentiviral transduction or using the CRISPR system with AAV6-mediated donor delivery (as depicted in the figure). The CAR-expressing CD34+ HSPCs are subsequently differentiated into SC-pDCs. The CAR SC-pDCs can either be injected directly into the patient to induce anti-tumour responses or be cryo-preserved for multiple vaccine regimens.

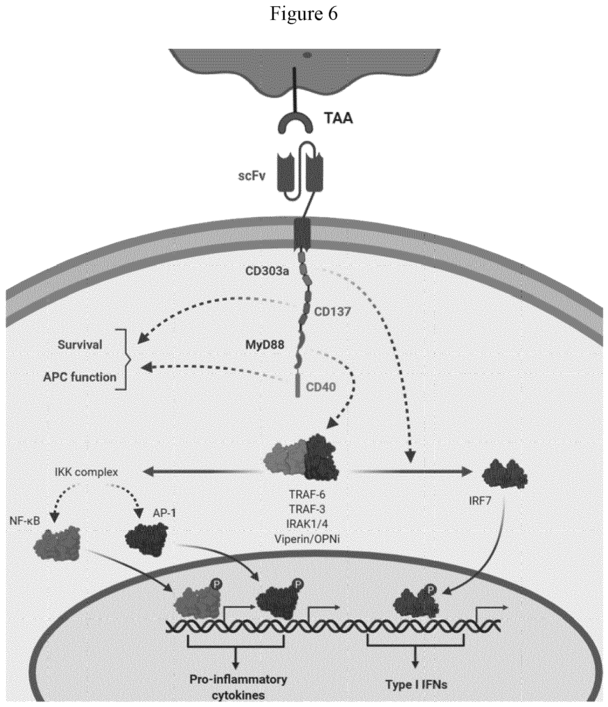

Figure 6 - **Different immune responses activated in SC-pDCs depending on the intracellular domain in the CAR.** Depending on the intracellular domain in the CAR, a wide variety of immunological responses can be activated achieved in SC-pDCs. Immunogenic intracellular domains (such as MyD88, CD137 and CD40) can induce activation of SC-pDCs and, for example, production of type I IFN or pro-inflammatory proteins, promote survival, promote antigen presentation. Also, tolerogenic intracellular domains (such as CD85g, PDL-1, ICOS-L) can induce tolerogenic functions of SC-pDCs, including tolerance of autoreactive T cells.

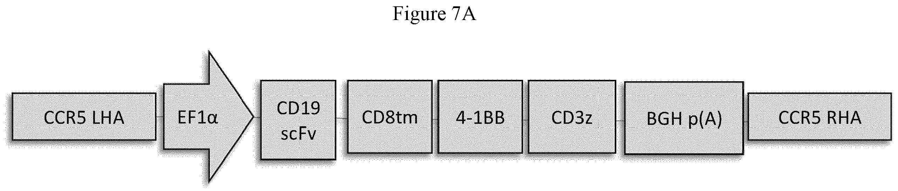

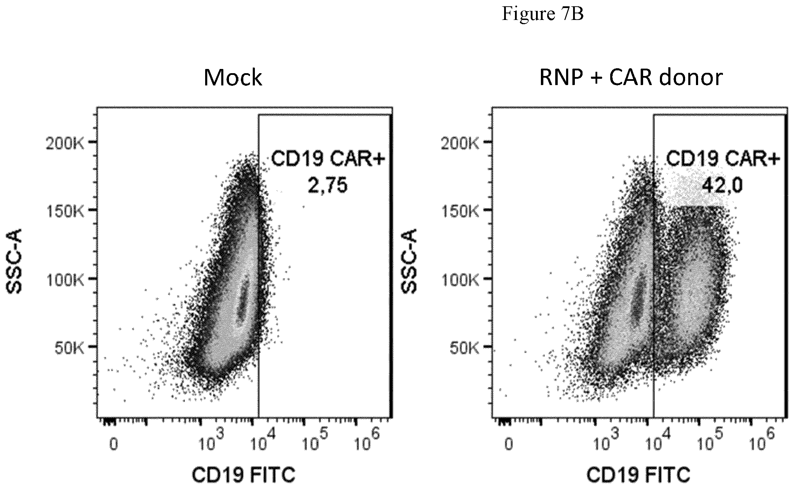

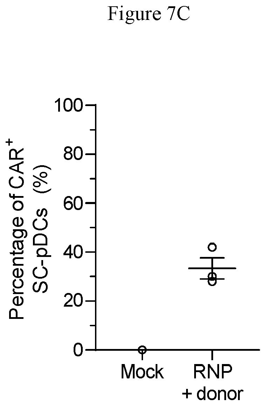

Figure 7 - **A conventional CAR can be expressed in SC-pDCs.** A) Schematic of a conventional 2nd generation anti-CD19 CAR. The construct contains the 4-1BB and CD3z intracellular domains. In T cells, expression of the CAR construct will allow the cells to elicit a T cell response upon binding to CD19. Moreover, homology arms directed against the safeharbour locus CCR5 flank the construct. This allows for the CAR to be safely inserted into this gene using CRISPR/Cas9-mediated homology directed repair (HDR).
   B) Representative flow plot showing expression of antiCD19 CAR in SC-pDCs. C) Column diagram showing percentage of antiCD19 CAR+ SC-pDCs for three donors.

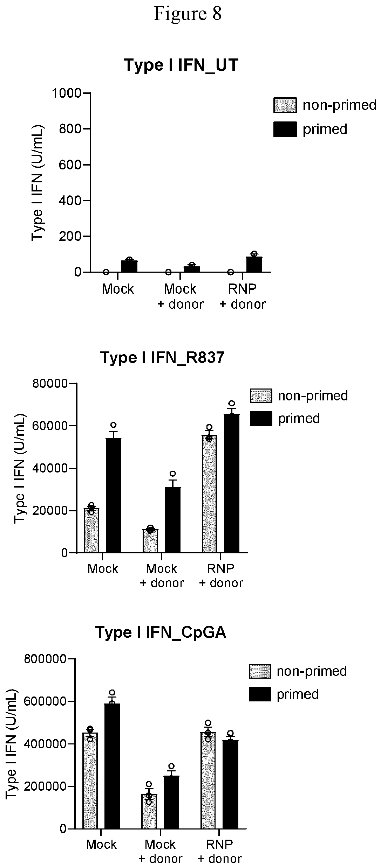

Figure 8 - **Type I IFN response of SC-pDCs genetically modified using CRISPR/Cas9 to express a CD19-CAR.**
   Primed SC-pDCs, where 30% of the cells express an anti-CD19 CAR (RNP+donor), or SC-pDCs not expressing the construct (mock + donor), were stimulated for 20 hours with agonists directed against TLR7 (R837), TLR9 (CpGA) or remained unstimulated (UT).
   Supernatants were subsequently harvested and bioactive type I IFN was evaluated using a commercially-available type I IFN bioassay (HEK-blue Type I IFN reporter bioassay).

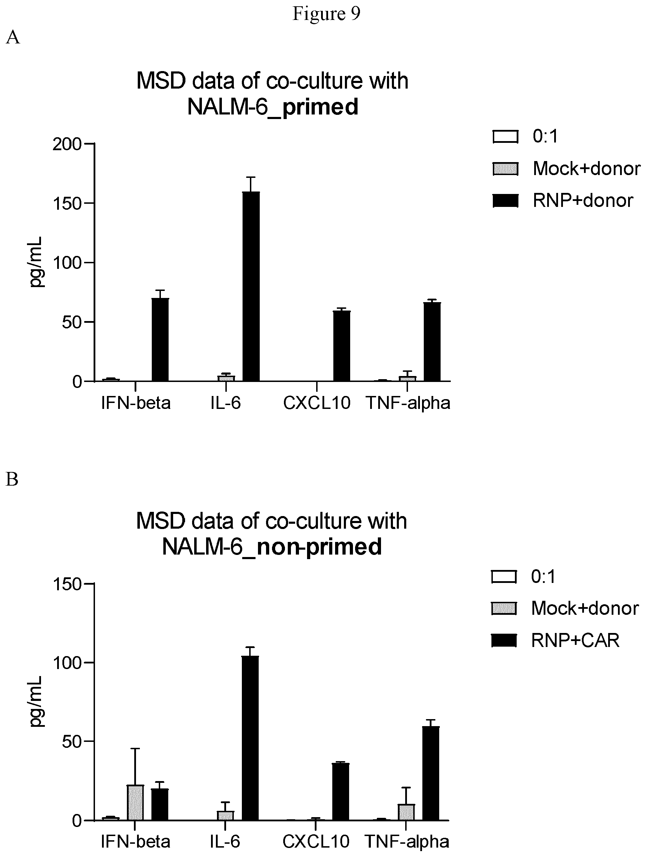

Figure 9 - **Cytokine response of SC-pDCs modified to express a CAR.**
   SC-pDCs primed for three days in medium supplemented with type I and II IFN (A) or left non-primed (B). Cells were subsequently co-cultured with the CD19+ target cell line (NALM-6) at an effector:target ratio of 1:1. Target cells were also seeded without effector cells (0: 1) to exclude potential background. After 20 hours, supernatants were harvested and levels of the cytokines IFN-beta, IL-6, CXCL10 and TNF-alpha were quantified using a Mesoscale multiplex cytokine assay (MSD). Data from one donor.

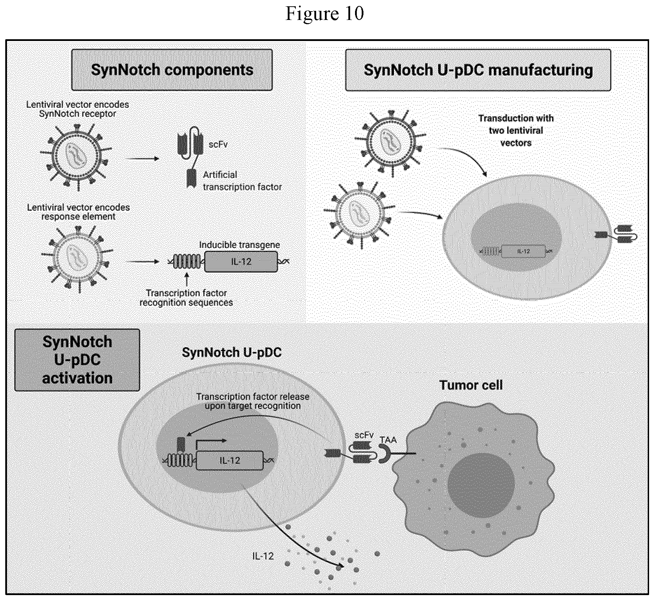

Figure 10 - **Schematic showing the generation of synNotch SC-pDCs by lenti-viral co-transduction of the synNotch receptor and synNotch response element into hematopoietic stem and progenitor cells (HSPCs), followed by differentiation of the stem cells into SC-pDCs.** Upon recognition of a target cell by the SynNotch SC-pDCs (by binding of the synNotch receptor to its cognate antigen), the SynNotch SC-pDCs will become activated, resulting in the transcription of a specific gene of interest, for example interleukin 12 (IL-12). "U-pDC" is an alternative term that is also used to refer to SC-pDCs.

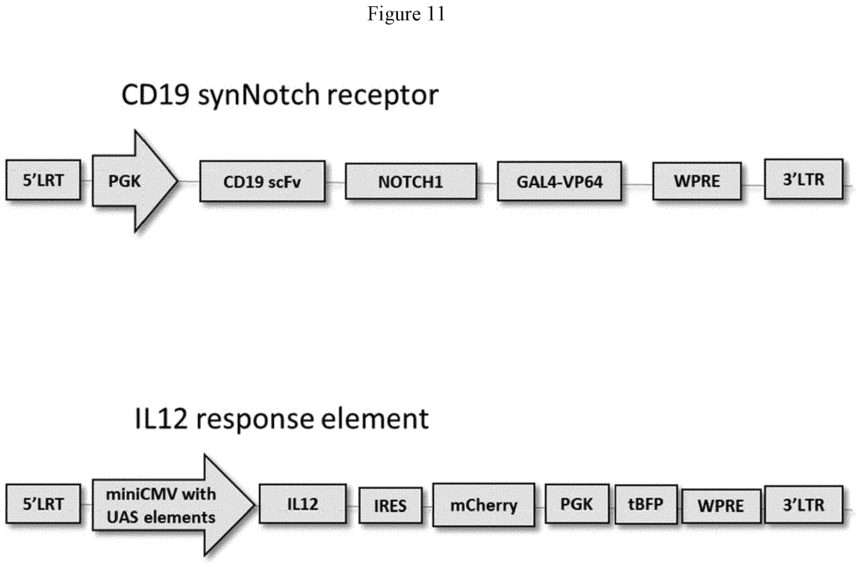

Figure 11 - **Schematic showing the synNotch receptor targeting CD19 and the IL-12 response element**

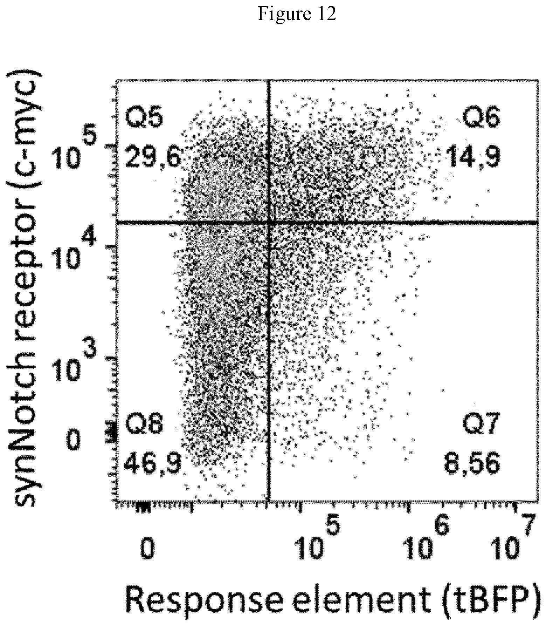

Figure 12 - **Expression of the response element on SC-pDCs.** Expression levels of SynNotch receptor and SynNotch response element in SC-pDCs. "U-pDC" is an alternative term that is also used to refer to SC-pDCs.

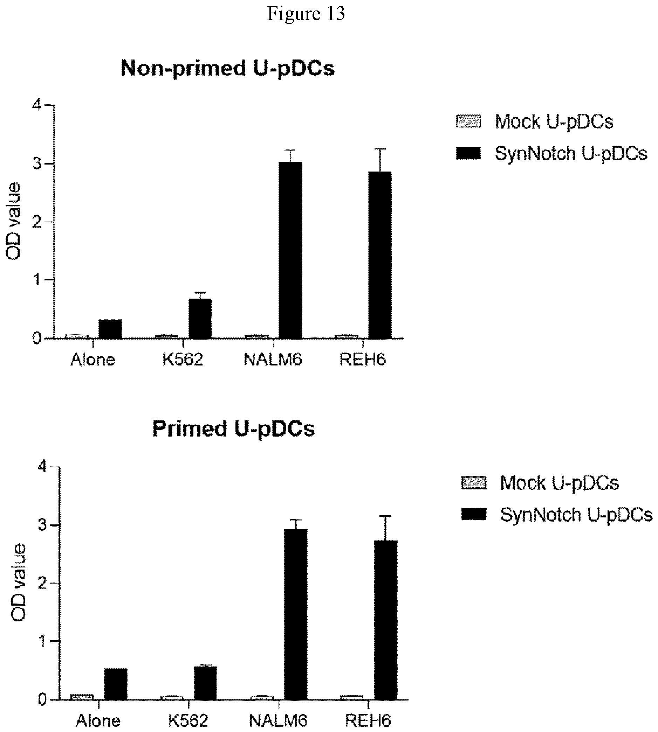

Figure 13 - **SynNotch pDCs bind target cells resulting in IL-12 secretion.** Co-culture of primed or non-primed synNotch SC-pDCs with target cells and analysis of synNotch receptor activity (IL12 secretion). SynNotch SC-pDCs (primed or non-primed) were co-cultured with CD19+ target cells (NALM6 or REH6) or CD 19-negative non-target cells (K562), or cultivated without cells (alone). Target:effector cell ratio was 5:1 (5x more target cells). Supernatants were collected 24 hours later and activation of the synNotch response element was assessed by analysing levels of IL-12 by ELISA. "U-pDC" is an alternative term that is also used to refer to SC-pDCs.

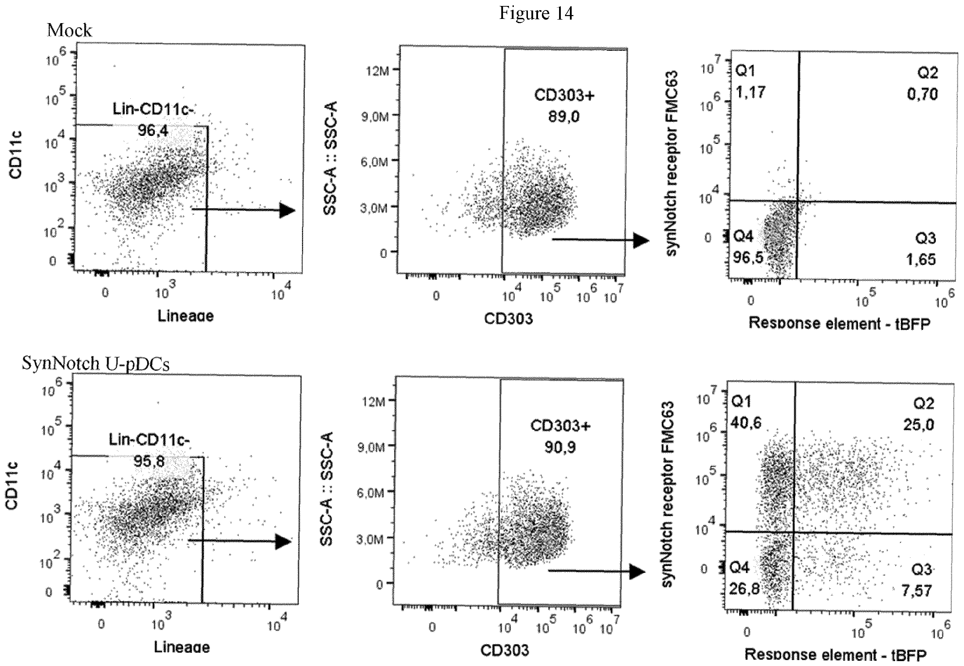

Figure 14 - **Expression of synNotch receptor and response element on primed SC-pDCs.** HSPCs were thawed and pre-expanded at low density. After 3 days of culture, HSPCs were co-transduced with SynNotch constructs and subsequently differentiated into SC-pDCs. After 16 days of culture, pDCs were isolated and primed before expression of synNotch receptor and response element was assessed on pDCs (lineage negative, CD11c negative, CD303+ cells). Data shown are for one donor. "U-pDC" is an alternative term that is also used to refer to SC-pDCs.

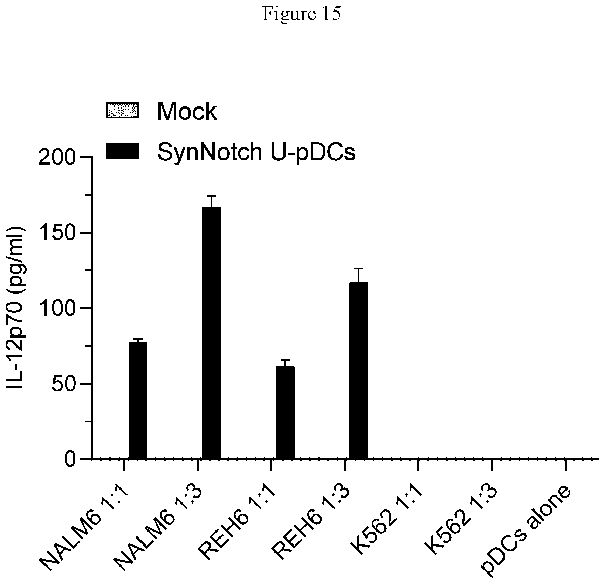

Figure 15 - **Activation of SC-pDCs upon recognition of CD19+ tartet cells.** 100.000 primed SC-pDCs were co-cultured with CD19+ NALM6 or REH6 target cells, or the non-target cells K562 (control), at a ratio of 1:1 and 1:3 effector: target. Supernatants were collected 20 hours after stimulation and levels of IL12 was analyzed using ELISA. Data shown are for one donor and biological triplicates. "U-pDC" is an alternative term that is also used to refer to SC-pDCs.

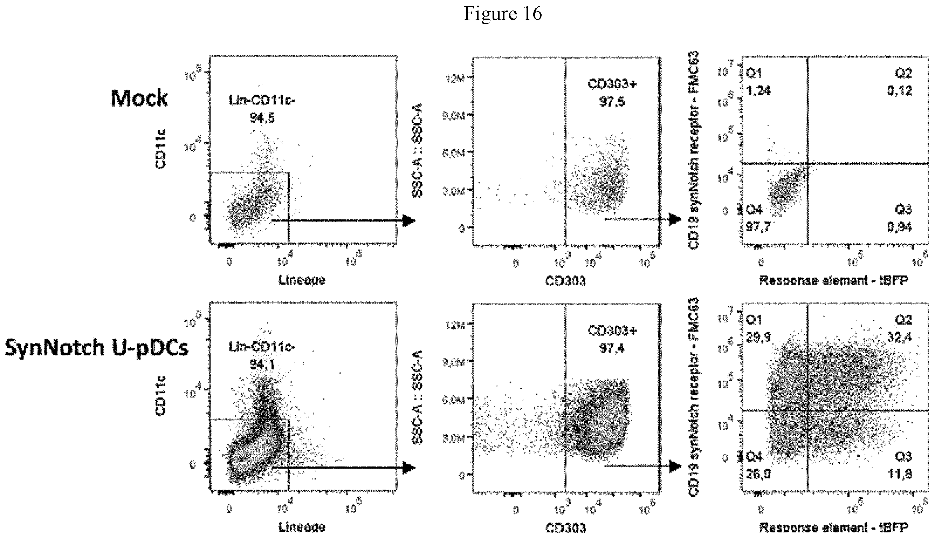

Figure 16 - **Expression of synNotch receptor and response element on primed SC-pDCs**. HSPCs were thawed and pre-expanded at low density. After 3 days of culture, HSPCs were co-transduced with SynNotch constructs and subsequently differentiated into SC-pDCs. After 16 days of culture, SC-pDCs were isolated and primed before expression of synNotch receptor and response element was assessed on SC-pDCs (lineage negative, CD11c negative, CD303+ cells). Data shown are for one donor. "U-pDC" is an alternative term that is also used to refer to SC-pDCs.

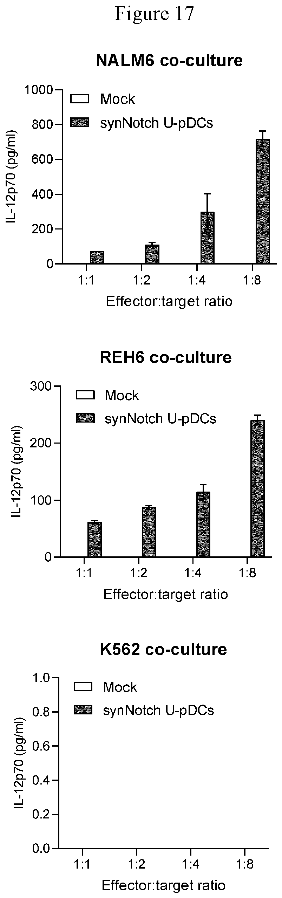

Figure 17 - **Activation of SC-pDCs upon recognition of CD19+ target cells.** 100.000 primed SC-pDCs were co-cultured with CD19+ NALM6 or REH6 target cells, or the non-target cells K562 (control), at a ratio of 1:1, 1:2, 1:4 and 1:8 effector: target. Supernatants were collected 20 hours after stimulation and levels of IL12 was analyzed using ELISA. Data shown are for one donor and biological triplicates. "U-pDC" is an alternative term that is also used to refer to SC-pDCs.

### Detailed Description of the Invention

### Constructs comprising extracellular antigen recognition domains

The invention provides engineered plasmacytoid dendritic cells that express on their surface an exogenous construct comprising an extracellular antigen recognition domain that recognises an antigen on a target cell and of use of such cells to treat disease, wherein the cell activates an immune response when the extracellular antigen recognition domain binds the target cell. The exogenous constructs allow the cells to recognise and bind specific target cells and to activate immune responses on binding. In preferred embodiments, the exogenous construct further comprises a transmembrane domain and an intracellular signalling domain that activates an immune response in the cell when the extracellular antigen recognition domain binds the target cell.

In some embodiments, the exogenous construct is a viral construct. In some embodiments, the viral construct is an AAV construct, an adenoviral construct, a lentiviral construct, or a retroviral construct.

In some embodiments, the exogenous construct is integrated into the genome of the engineered cell. In some embodiments, the exogenous construct is not integrated into the genome of the engineered cell. In some embodiments, the exogenous construct is introduced by a transposase, retrotransposase, episomal plasmid, mRNA, or random integration. Preferably, the exogenous construct is introduced with a gene editing system such as TALEN, zinc finger or, most preferably, CRISPR/Cas9. In further preferred embodiments, the exogenous construct is introduced through transduction with a viral vector, such as a lentiviral vector.

The term exogenous as used herein has its normal meaning. In particular, the exogenous construct is a construct that has been introduced into the cell and that is not present in an unmodified cell in the same configuration or location. The exogenous construct may be constructed using endogenous (preferably human) sequences.

Each domain may be heterogeneous, that is, comprised of sequences derived from different protein chains.

The extracellular antigen recognition domain recognises an antigen on a target cell and can be designed or selected according to the desired therapeutic use or target cell. In some embodiments, the antigen recognition domain is an antibody or fragment thereof, e.g., a Fab, Fab', Fv, F(ab')2, dAb, or preferably, one or more single-domain antibodies ("nanobodies") or one or more single chain antibody fragments ("scFv"). A scFv is a single chain antibody fragment having the variable regions of the heavy and light chains of an antibody linked together. A single-domain antibody, also known as a nanobody, is an antibody fragment consisting of a single monomeric variable antibody domain. scFvs and nanobodies are useful in chimeric antigen receptors because they may be engineered to be expressed as part of a single chain along with the other CAR components.

The extracellular antigen recognition domain will generally recognise a cell surface antigen. In certain embodiments, for example when used for treating cancer, the extracellular antigen recognition domain will recognise and specifically bind a tumour associated antigen, which is an antigen that is expressed exclusively on tumour cells or that is expressed at a higher level by tumour cells relative to healthy cells. In certain embodiments, for example when used for treating an autoimmune disease, the extracellular antigen recognition domain will recognise and specifically bind an autoantigen (or self-antigen) that is targeted by the pathogenic autoreactive immune cells underlying the autoimmune disease. In certain embodiments, for example when used for treating an inflammatory disease, the extracellular antigen recognition domain will recognise and specifically bind an antigen that is tissue-specific and expressed on the surface of cells of a particular tissue, such a tissue that is inflamed, a transplanted tissue, or tissue that is damaged or at risk of damage from inflammation.

In certain embodiments, the extracellular antigen recognition is bispecific or multispecific, with specificity to more than one target of interest.

The extracellular antigen recognition domain recognises an antigen on a target cell. The antigen may therefore be a target antigen. In certain embodiments, the domain specifically binds the antigen and does exhibit significant binding to any other antigens, or exhibits significantly stronger binding to the target antigen than any other antigen.

In preferred embodiments, the antigen recognised by the extracellular antigen recognition domain is a tumour antigen, preferably a tumour-associated surface antigen. In certain embodiments, the tumour antigen is selected from the group consisting of 5T4, alphafetoprotein (AFP), B7-1 (CD80), B7-2 (CD86), BCMA, B-human chorionic gonadotropin, CA-125, carcinoembryonic antigen (CEA), carcinoembryonic antigen (CEA), CD123, CD133, CD138, CD19, CD20, CD22, CD23, CD24, CD25, CD30, CD33, CD34, CD4, CD40, CD44, CD56, CD70, CD8, CLL-l, c-Met, CMV-specific antigen, CS-l, CSPG4, CTLA-4, DLL3, disialoganglioside GD2, ductal -epithelial mucine, EBV-specific antigen, EGFR variant III (EGFRvIII), ELF2M, endoglin, ephrin B2, epidermal growth factor receptor (EGFR), epithelial cell adhesion molecule (EpCAM), epithelial tumour antigen, ErbB2 (HER2/neu), fibroblast associated protein (fap), FLT3, folate binding protein, GD2, GD3, glioma-associated antigen, glycosphingolipids, gp36, HBV- specific antigen, HCV-specific antigen, HER1, HER2, HER2-HER3 in combination, HERV-K, high molecular weight-melanoma associated antigen (HMW-MAA), HIV-l envelope glycoprotein gp4l, HPV-specific antigen, human telomerase reverse transcriptase, IGFI receptor, IGF-II, IL-11Ralpha, IL-13R-a2, Influenza Virus-specific antigen, CD38, insulin growth factor (IGF1)-l, intestinal carboxyl esterase, kappa chain, LAGA-la, lambda chain, Lassa Virus-specific antigen, lectin-reactive AFP, lineage-specific or tissue specific antigen such as CD3, MAGE, MAGE-A1, major histocompatibility complex (MHC) molecule, major histocompatibility complex (MHC) molecule presenting a tumour-specific peptide epitope, M-CSF, melanomaassociated antigen, mesothelin, MN-CA IX, MUC-l, mut hsp70-2, mutated p53, mutated p53, mutated ras, neutrophil elastase, NKG2D, Nkp30, NY-ESO-1, p53, PAP, prostase, prostate specific antigen (PSA), prostate-carcinoma tumour antigen- 1 (PCTA-l), prostate-specific antigen protein, STEAP1, STEAP2, PSMA, RAGE-l, ROR1, RU1, RU2 (AS), surface adhesion molecule, survivin, telomerase, TAG-72, the extra domain A (EDA) and extra domain B (EDB) of fibronectin and the A1 domain of tenascin-C (TnC Al), thyroglobulin, tumour stromal antigens, vascular endothelial growth factor receptor-2 (VEGFR2), virus-specific surface antigen such as an HIV-specific antigen (such as HIV gpl20), as well as any derivate or variant of these surface markers.

In preferred embodiments, the extracellular antigen recognition domain recognises an antigen selected from the group consisting of BCMA, CD19, CLL1, CS1, STEAP1, STEAP2, CD70, and CD20. In some embodiments, the extracellular antigen recognition domain specifically targets CD19.

In further preferred embodiments, the antigen recognised by the extracellular antigen recognition domain is an autoantigen associated with the autoimmune disease to be treated. Exemplary antigens to be targeted include (with the associated disease to be treated in parenthesis): GAD65 (type 1 diabetes), insulinoma associated protein 2 - IA-2 (type 1 diabetes), thyroid peroxidase - TPO (thyroid autoimmune diseases, Hashimoto's and Graves'), thyrotropin receptor - TSHR (thyroid autoimmune diseases, Hashimoto's and Graves'), adrenocorticotropic hormone receptor - ACTHR (Addison's adrenal insufficiency), 21-hydroxylase (Addison's adrenal insufficiency), 17-alpha-hydroxylase (oophoritis), P450 side-chain cleavage enzyme (oophoritis), sperm antigens (orchitis), pituitary cytosolic protein (lymphocytic hypophysitis), calcium sensing receptor - CaSR (autoimmune hypoparathyroidism), NACHT LRR and PYD domains-containing protein 5 - NALP5 (autoimmune hypoparathyroidism), alpha 3 chain of type IV collagen (Goodpasture's disease), myosin (Autoimmune myocarditis), Type-M phospholipase A2 receptor - PLA2R (membranous nephropathy), Cytochrome P450 1A2 (autoimmune hepatitis), tropomyosin isoform 5 - hTM5 (ulcerative colitis), major zymogen granule membrane glycoprotein 2 - GP2 (Crohn's disease), myelin basic protein - MBP (multiple sclerosis), myelin oligodendrocyte glycoprotein -MOG (multiple sclerosis), nicotinic acetylcholine receptor - AChR (myasthenia gravis), aquaporin-4 - AQP4 (neuromyelitis optica).

When used to treat inflammatory disease, the extracellular antigen recognition domain in the exogenous construct is selected to target the afflicted organ, such as pancreas or islet of Langerhans, kidney, heart, lung, liver, intestine, skin or joints. Exemplary antigens to be targeted include (with the associated organ in parenthesis): GAD65 (pancreas or islet of Langerhans), insulinoma associated protein 2 - IA-2, (pancreas or islet of Langerhans), type-M phospholipase A2 receptor - PLA2R (kidney), myosin (heart), alpha 3 chain of type IV collagen (lung), cytochrome P450 1A2 (liver), major zymogen granule membrane glycoprotein 2 - GP2 (intestine), tropomyosin isoform 5 - hTM5 (intestine), carcinoembryonic antigen - CEA (intestine).

When used to treat transplant rejection, the extracellular antigen recognition domain in the exogenous construct is selected to target the afflicted organ, such as pancreas or islet of Langerhans, kidney, heart, lung, liver or intestine. Exemplary antigens to be targeted include (with the associated organ in parenthesis): GAD65 (pancreas or islet of Langerhans), insulinoma associated protein 2 - IA-2, (pancreas or islet of Langerhans), type-M phospholipase A2 receptor - PLA2R (kidney), myosin (heart), alpha 3 chain of type IV collagen (lung), cytochrome P450 1A2 (liver), major zymogen granule membrane glycoprotein 2 - GP2 (intestine), tropomyosin isoform 5 - hTM5 (intestine), carcinoembryonic antigen - CEA (intestine). Alternatively, the extracellular antigen recognition domain in the exogenous construct may target HLA molecules, which could be particularly effective in the context of HLA-disparate transplantation, because the extracellular antigen recognition domain will recognise only the transplanted organ.

The intracellular signalling domain activates an immune response in the cell when the extracellular antigen recognition domain binds the target cell. The immune response may be inflammatory or it may be anti-inflammatory, depending on the desired therapeutic use.

In preferred embodiments, the immune response activated in the cell is the secretion of cytokines. Depending on the desired therapeutic effect and context, cytokines may include homeostatic cytokines, chemokines, pro-inflammatory cytokines, anti-inflammatory cytokines, effectors, and/or acute-phase proteins. For example, homeostatic cytokines, including interleukin (IL) 7 and IL-15, promote immune cell survival and proliferation, and pro-inflammatory cytokines may promote an inflammatory response, which may be useful in cancer therapy. Examples of homeostatic cytokines include, but are not limited to, IL-2, IL-4, IL-5, IL-7, IL- 10, IL-12p40, IL-12p70, IL-15, and interferon (IFN) gamma. Examples of pro-inflammatory cytokines include, but are not limited to, IL-1a, IL-1b, IL-6, IL-13, IL-17a, tumour necrosis factor (TNF)-alpha, TNF-beta, fibroblast growth factor (FGF) 2, granulocyte macrophage colony-stimulating factor (GM-CSF), soluble intercellular adhesion molecule 1 (sICAM-l), soluble vascular adhesion molecule 1 (sVCAM-1), vascular endothelial growth factor (VEGF), VEGF-C, VEGF-D, and placental growth factor (PLGF). Examples of effectors include, but are not limited to, granzyme A, granzyme B, soluble Fas ligand (sFasL), and perforin. Examples of acute phase-proteins include, but are not limited to, C - reactive protein (CRP) and serum amyloid A (SAA). Preferred cytokines expressed following activation of the intracellular signalling domain include IFNα, IFNβ, IFNλ, TNFα, IL-6, CXCL9, CXCL10 and/or CXCL11.

In certain embodiments, the activated immune response comprises TRAIL-mediated killing.

In certain embodiments, the activated immune response comprises expression of checkpoint inhibitors.

In certain embodiments, the intracellular signalling domain comprises one or more stimulatory or co-stimulatory domains derived from a molecule selected from the group consisting of 4-1BB/CD137, B7-H3, BAFFR, BLAME (SLAMF8), BTLA, CD33, CD45, CD100 (SEMA4D), CD103, CD134, CD137, CD154, CD16, CD 160 (BY55), CD18, CD19, CD19a, CD2, CD22, CD247, CD27, CD276 (B7-H3), CD28, CD29, CD3 (alpha; beta; delta; epsilon; gamma; zeta), CD30, CD37, CD4, CD4, CD40, CD49a, CD49D, CD49f, CD5, CD64, CD69, CD7, CD80, CD83 ligand, CD84, CD86, CD 8 alpha, CD8beta, CD9, CD96 (Tactile), CD1-1a, CD1-1b, CD1-1c, GDI -Id, CDS, CEACAM1, CRT AM, DAP-10, DNAM1 (CD226), Fc gamma receptor, GADS, GITR, HVEM (LIGHTR), IA4, ICAM-l , ICAM-l, ICOS, Ig alpha (CD79a), IL2R beta, IL2R gamma, IL7R alpha, integrin, 1TGA4, 1TGA4, 1TGA6, 1TGAD, ITGAE, ITGAL, ITGAM, ITGAX, ITGB2, ITGB7, ITGB1, KIRDS2, LAT, LFA-1, LFA-l, LIGHT, LIGHT (tumour necrosis factor superfamily member 14; TNFSF14), LTBR, Ly9 (CD229), lymphocyte function-associated antigen- 1 (LFA-l (CD1 la/CD 18), MHC class I molecule, NKG2C, NKG2D, NKp30, NKp44, NKp46, NKp80 (KLRF1), 0X40, PAG/Cbp, PD-l, PSGL1, SELPLG (CD 162), signalling lymphocytic activation molecule, SLAM (SLAMF1; CD150; IPO-3), SLAMF4 (CD244; 2B4), SLAMF6 (NTB-A; Lyl08), SLAMF7, SLP-76, TNF, TNFr, TNFR2, Toll ligand receptor, TRANCE/RANKL, VLA1, and VLA-6, or fragments, truncations, or combinations thereof. Intracellular signalling domain comprising the above domains will be particularly useful for treating cancer.

Preferred intracellular signalling domains include CD3z, CD40, MyD88 (ΔTIR), MyD88, CD300a, CD300c, CD137 (4-1BB), DAP-10, DAP-12, FCGR2, FCERG, TLR1 (TIR), TLR7 (TIR), TLR9 (TIR), CD335 (NKp46), CD158 (KIR2DL), CD19 and FKBPv36 (inducible)..

In certain embodiments, in particular for the treatment of autoimmune diseases and inflammatory diseases, the intracellular signalling domain activates a tolerogenic or anti-inflammatory response. In such embodiments, the intracellular signalling domain may comprise a domain derived from a molecule selected from the group consisting of CD85g, CD275, CD279, CD303, CD305 and IL10R.

In certain embodiments, the immune response that is activated is the recruitment of immune cells, such as Tregs (in particular for treating autoimmune and inflammatory disease) or cytotoxic lymphocytes (in particular for treating cancer).

In preferred embodiments, the exogenous construct is a chimeric antigen receptor (CAR). CARs comprise an extracellular antigen recognition domain, which is generally an scFv with specificity for a particular cell surface antigen, such as a tumour antigen, and which is directly or indirectly linked to an intracellular signalling domain. The intracellular signalling domain may comprise separate domains, such as one or more costimulatory domains and/or one or more activating domains. The components may be arranged in any order. In some embodiments, a CAR is engineered such that the costimulatory domain is expressed as a separate polypeptide chain. Preferred CARs comprise extracellular antigen recognition domains and intracellular signalling domains as described above.

In further preferred embodiments, the exogenous construct is a synthetic Notch (synNotch) receptor. SynNotch receptors comprise an extracellular antigen recognition domain, which may be an scFv with specificity for a particular cell surface antigen, such as a tumour antigen, a transmembrane domain, which controls proteolysis and cleavage, and a intracellular signalling domain, which may be a transcription factor. Binding of the antigen recognition domain releases the transcription factor, which can target a response element with recognition elements for the transcription factor and drive expression of a gene or set of genes, thereby activating an immune response. SynNotch receptors allow the immune response activated by the construct to be customised for the desired therapeutic effect by driving expression of any gene or set of genes. The system can also be multiplexed with multiple transcription factor domains and/or response elements.

In embodiments wherein the exogenous construct is a synthetic Notch (synNotch) receptor, the engineered cell generally also comprises a response element that is activated by the intracellular signalling domain, which is preferably a transcription factor (such as, for example, Gal4-VP64, Gal4-VPl6, TetR-VP64, LacI-VP64, and the like, or such as Gal4-VP64, Gal4-VPl6, Gal4-VPR, TetR-VP64, LacI-VP64, and the like). A preferred transcription factor is Gal4-VP64. The response element is operably linked to a gene that mediates the immune response activated by the construct. The response element and gene may be integrated in the genome or present on a vector such as a plasmid. The response element and gene may be introduced by any appropriate technique, such as by transposase, retrotransposase, episomal plasmid or random integration. Preferably, the response element is introduced with a gene editing system such as TALEN, zinc finger or, most preferably, CRISPR/Cas9. In further preferred embodiments, the response element is introduced by lentiviral transduction. Preferred synNotch receptors comprise extracellular antigen recognition domains described above. Preferred synNotch receptors comprise intracellular signalling domains, such as transcription factors, that activate expression of the cytokines described above, or the stimulatory molecules described above. In certain embodiments, the response element encodes TRAIL, a checkpoint inhibitor, such as PDL1, IL-12 or Type I IFN. In preferred embodiments, expression of PD-L1 or IL10 is activated. In further preferred embodiments, expression of IL12 is activated.

A preferred Notch gene is NOTCH1. Alternative Notch genes include NOTCH2, NOTCH3 and NOTCH4.

The exogenous construct comprises a transmembrane domain linking the extracellular antigen recognition domain and the intracellular signalling domain. The transmembrane domain may be derived from the transmembrane domain of 4-1BB/CD137, an alpha chain of a T cell receptor, a beta chain of a T cell receptor, a gamma chain of a T cell receptor, a delta chain of a T cell receptor, CD3 epsilon, CD3 delta, CD3 gamma, CD3 zeta, CD4, CD5, CD8 alpha, CD9, CD16, CD19, CD22, CD33, CD34, CD37, CD45, CD64, CD80, CD86, CD 134, CD137, CD154, or a zeta chain of a T cell receptor, or any combination thereof.

In certain embodiments, the exogenous construct comprises a hinge or spacer, in particular to allow free movement of the extracellular antigen recognition domain. Suitable hinge or spacers include regions of IgG1, IgG2, IgG3, IgG4, IgA, IgD, IgE, and IgM, or a fragment thereof. In some embodiments, the hinge or spacer is from CD8 alpha. Optionally, short linkers may form linkages between any or some of the extracellular, transmembrane, and intracellular domains of the construct. In some embodiments, the linker may be derived from repeats of glycine-glycine-glycine-glycine-serine. The linkers may also be used as a peptide tag. The linker peptide sequence may be of any appropriate length to connect one or more proteins of interest and is preferably designed to be sufficiently flexible so as to allow the proper folding and/or function and/or activity of one or both of the peptides it connects.

In preferred embodiments, the exogenous construct is a chimeric antigen receptor comprising:
a) an extracellular antigen recognition domain that is an anti-CD19 scFv,
b) a CD8 transmembrane domain,
c) a 4-1BB intracellular signalling domain,
d) a CD3z intracellular signalling domain, and
e) a BGH poly(A) intracellular signalling domain,
   or two or more of (a)-(e), such as three or four of (a)-(e).

In further preferred embodiments, the exogenous construct is a synNotch receptor comprising:
a) a promoter, such as PGK,
b) an extracellular antigen recognition domain that is an anti-CD19 scFv,
c) NOTCH1
d) a Gal4-VP64 transcription factor, and
e) optionally a Woodchuck Hepatitis Virus (WHV) Posttranscriptional Regulatory Element (WPRE);
and the engineered cell also comprises a response element comprising:
a) a promoter responsive to the transcription factor in the synNotch receptor such as the minimal CMV promoter preceded by 5x UAS elements, which are target sequences for Gal4,
b) IL-12,
c) an IRES,
d) a marker, such as mCherry,
e) a promoter, such as PGK, followed by a reporter, such as tBFP, and
f) optionally a Woodchuck Hepatitis Virus (WHV) Posttranscriptional Regulatory Element (WPRE).

The Examples demonstrate that such constructs can be expressed by pDCs, which maintain their functionality and type IIFN response, and can be used to successfully recognise target cells, bind and subsequently become activated to produce IFN-beta and proinflammatory factors.

In certain embodiments, the exogenous construct comprises the extracellular antigen recognition domain and does not comprise an intracellular signalling domain. Such constructs may or may not contain transmembrane domains. In certain such embodiments, the extracellular antigen recognition domain is a single chain antibody fragment (scFv) or a recombinant T cell receptor. In such embodiments the engineered cell is targeted to a disease location and activates an immune response. pDCs area known to activate, expand and recruit Tregs, for example, which will be useful in the treatment of inflammatory diseases, autoimmune diseases and transplant rejection. The response may be mediated by TCR signalling, for example by CD3ζ.

In certain embodiments, the exogenous construct is a recombinant T cell receptor (TCR). TCRs are antigen-specific molecules that are responsible for recognizing antigenic peptides presented in the context of a product of the major histocompatibility complex (MHC) on the surface of antigen presenting cells or any nucleated cell (e.g., all human cells in the body, except red blood cells). In contrast, antibodies typically recognize soluble or cell-surface antigens, and do not require presentation of the antigen by an MHC. This system endows T-cells, via their TCRs, with the potential ability to recognize the entire array of intracellular antigens expressed by a cell (including virus proteins) that are processed intracellularly into short peptides, bound to an intracellular MHC molecule, and delivered to the surface as a peptide-MHC complex. In such embodiments, the immune response activated by the pDC on binding of the target may be mediated by endogenous TCR signalling pathways.

### Plasmacytoid dendritic cells (pDCs) for treating disease

The engineered cells for use in the invention are plasmacytoid dendritic cells (pDCs). Plasmacytoid dendritic cells (pDCs) have a multifaceted role in the immune system, which makes them extremely adaptable for the targeted treatments of the invention. pDCs are key effectors in cellular immunity with the ability to not only initiate immune responses but also to induce tolerance to exogenous and endogenous antigens (Swiecki, and Colonna, Nat Rev Immunol, 2015. 15(8)). pDCs are distinct from conventional DCs as their final stage of development occurs within the bone marrow; their antigens are taken up by receptormediated endocytosis; they express high levels of interferon regulatory factor 7; and they primarily sense pathogens through toll-like receptor (TLR) 7 and 9 (Swiecki and Colonna, Nat Rev Immunol, 2015. 15(8); and Tangand Cattral, Cell Mol Life Sci, 2016). Through these pattern-recognition receptors, pathogen nucleic acids can activate pDCs to produce high levels of type I interferon (IFN). Thus, activated pDCs link the innate and adaptive immune system together via cytokine production combined with antigen-presenting cell (APC) activity. Furthermore, pDC functionality is also essential to achieve an antiviral state during infections, provide vital adjuvant activity in the context of vaccination, and for promoting immunogenic anti-tumour responses upon activation (Swiecki, and Colonna, Nat Rev Immunol, 2015. 15(8); Tovey, et al. Biol Chem, 2008. 389(5); and Rajagopal, et al. Blood, 2010. 115(10): p. 1949-57). A delicate balance must be maintained, however, as hyperactivation of pDCs has been associated with the pathogenesis of several diseases, including viral infections, autoimmune diseases and tumourigenesis (Swiecki and Colonna, Nat Rev Immunol, 2015. 15(8); and Tang and Cattral, Cell Mol Life Sci, 2016).

Unlike T cells, NK cells, macrophages and other cells conventionally used with CAR constructs to treat cancer and autoimmune diseases, the therapeutic effects of the engineered pDCs of the invention are significantly different and they utilise the ability of pDCs to indirectly activate immune responses by releasing cytokines and activating other immune cells.

Preferably, the engineered pDCs express TRAIL. In another embodiment said pDCs express CD123, CD303, CD304, CD4 and/or HLA-DR. In yet another embodiment said pDCs express IFN type I, IFN type II, IFN type III and/or proinflammatory cytokines. In a further embodiment said pDCs express IRF7, TLR7 and/or TLR9. In one embodiment said pDCs express CD40, CD80, CD83 and/or CD86. For example, said pDCs express TRAIL, CD123, CD303, CD304, CD4, HLA-DR, IFN type I, IFN type II, IFN type III, IRF7, TLR7 and/or TLR9.

In one preferred embodiment of the present invention, the pDCs express TNF-related apoptosis-inducing ligand (TRAIL). TRAIL, which is also designated CD253, is a ligand involved in killing cancer cells by interacting with TRAIL receptors (death receptor 5, DR5) on the surface of cancer cells and thereby triggering apoptosis.

The engineered pDCs of the invention are preferably matured cells having a surface phenotype that strongly resembles blood pDCs. In a preferred embodiment, said pDCs express CD123, CD303, CD304, CD4 and/or HLA-DR.

In another preferred embodiment said pDCs express IFN type I, IFN type II, IFN type III and/or proinflammatory cytokines.

The pDCs may in one preferred embodiment express Toll-like receptors, such as for example Toll-like receptor 7 (TLR7) and/or Toll-like receptor 9 (TLR9).

In yet another preferred embodiment said pDCs express Interferon regulatory factor 7 (IRF7).

In a preferred embodiment said pDCs secretes IL-6.

In preferred embodiments, the engineered plasmacytoid dendritic cell is capable of a type I IFN response.

In another preferred embodiment said pDCs express Cluster of differentiation 80 (CD80), which is a protein found on Dendritic cells, activated B cells and monocytes that provides a costimulatory signal necessary for T cell activation and survival.

The pDCs may in a preferred embodiment also express proteins characteristic for antigen presenting cells such as for example Cluster of Differentiation 86 (CD86) and/or Cluster of Differentiation 40 (CD40). CD86 is a protein expressed on antigen-presenting cells that provides costimulatory signals necessary for T cell activation and survival, whereas CD40 is a costimulatory protein found on antigen presenting cells and is required for their activation.

Preferably, said pDCs express CD40, CD80, CD83 and/or CD86. In another preferred embodiment said pDCs express interleukin 6 (IL-6).

In preferred embodiments, the engineered pDCs of the invention are stem cell-derived plasmacytoid dendritic cell.

In certain embodiments, pDCs are autologous. Such treatments may minimise any risk of rejection of the transferred cells. In alternative embodiments, the cells are allogenic, such as isolated from healthy donors. Such treatments can potentially be prepared more quickly and offered "off the shelf'. In certain embodiments, the cells are or have been cryopreserved. Moreover, the cells may be xenogeneic.

### Methods for treating disease

The disclosure provides an engineered cell for use in a method of treating a disease in a subject, wherein the method comprises administering the engineered cell to the subject, wherein the cell expresses on its surface an exogenous construct comprising an extracellular antigen recognition domain that recognises an antigen on a target cell, and wherein the cell activates an immune response when the extracellular antigen recognition domain binds the target cell. Therefore, the invention provides cells for a new adoptive cell therapy. Adoptive cell therapy is the transfer of *ex vivo* grown cells, most commonly immune-derived cells, into a host with the goal of transferring the immunologic functionality and characteristics of the transferred cells. Adoptive cell therapy is well established for treating cancer and autoimmune and inflammatory diseases, albeit using different transferred immune cells with different immune-regulating effects and activities.

In certain embodiments of the invention, the engineered cell is for use in methods of treatment that may comprise (i) collecting autologous hematopoietic stem progenitor cells (HSPCs), either from the subject to be treated or a healthy donor; (ii) preparing engineered pDCs, for example using a method discussed below; (iii) optionally administering to the subject lymphodepleting chemotherapy; and (iv) administering to the subject the engineered pDCs.

In certain embodiments, the engineered cell of the invention is for use in methods that may comprise administering cells expressing more than one exogenous construct. Individual cells may express more than one construct, or the population of cells administered may comprise a plurality of different cells.

In certain embodiments, the engineered cells are further modified to express immunemodulatory proteins, such as cytokines (e.g., IL-2, IL-12 or IL-15), which may stimulate T-cell activation and recruitment, and may thus aid in combating the tumour microenvironment. Thus, the cells may comprise a population of cells expressing the exogenous construct and further expressing an immune modulatory protein such as, for example, IL-2, IL-12, or IL-15.

In certain embodiments, the engineered cells may be isolated from a subject and used fresh, or frozen for later use, in conjunction with (e.g., before, simultaneously or following) lymphodepletion.

In certain embodiments, the engineered cells may be administered to the subject by dose fractionation, wherein a first percentage of a total dose is administered on a first day of treatment, a second percentage of the total dose is administered on a subsequent day of treatment, and optionally, a third percentage of the total dose is administered on a yet subsequent day of treatment.

An exemplary total dose comprises 10³ to 10¹¹ cells/kg body weight of the subject, such as 10³ to 10¹⁰ cells/kg body weight, or 10³ to 10⁹ cells/kg body weight of the subject, or 10³ to 10⁸ cells/kg body weight of the subject, or 10³ to 10⁷ cells/kg body weight of the subject, or 10³ to 10⁶ cells/kg body weight of the subject, or 10³ to 10⁵ cells/kg body weight of the subject. Moreover, an exemplary total dose comprises 10⁴ to 10¹¹ cells/kg body weight of the subject, such as 10⁵ to 10¹¹ cells/kg body weight, or 10⁶ to 10¹¹ cells/kg body weight of the subject, or 10⁷ to 10¹¹ cells/kg body weight of the subject.

An exemplary total dose may be administered based on a patient body surface area rather than the body weight. As such, the total dose may include 10³ to 10¹³ cells per m².

In certain embodiments of the invention, the engineered cell of the invention is for use in methods that comprise lymphodepletion. Lymphodepletion may be achieved by any appropriate means. Lymphodepletion may be performed prior to administration of the engineered cells, or subsequent to. In certain embodiments, lymphodepletion is performed both before and after administration of the engineered cells.

In certain embodiments of the invention, the engineered cell of the invention is for use in methods that may comprise administration of one or more additional therapeutic agents. Exemplary therapeutic agents include a chemotherapeutic agent, an anti-inflammatory agent, an immunosuppressive, an immunomodulatory agent, or a combination thereof.

Therapeutic agents may be administered according to any standard dose regime known in the field. Exemplary chemotherapeutic agents include anti-mitotic agent, such as taxanes, for instance docetaxel, and paclitaxel, and vinca alkaloids, for instance vindesine, vincristine, vinblastine, and vinorelbine. Exemplary chemotherapeutic agents include a topoisomerase inhibitor, such as topotecan. Exemplary chemotherapeutic agents include a growth factor inhibitor, a tyrosine kinase inhibitor, a histone deacetylase inhibitor, a P38a MAP kinase inhibitor, inhibitors of angiogenesis, neovascularization, and/or other vascularization, a colony stimulating factor, an erythropoietic agent, an anti-anergic agents, an immunosuppressive and/or immunomodulatory agent, a virus, viral proteins, immune checkpoint inhibitors, BCR inhibitors (e.g., BTK, P13K, etc.), immune-metabolic agents (e.g., IDO, arginase, glutaminase inhibitors, etc.), and the like. According to certain aspects of the present invention, the one or more therapeutic agents may comprise an antimyeloma agent. Exemplary antimyeloma agents include dexamethasone, melphalan, doxorubicin, bortezomib, lenalidomide, prednisone, carmustine, etoposide, cisplatin, vincristine, cyclophosphamide, and thalidomide, several of which are indicated above as chemotherapeutic agents, anti-inflammatory agents, or immunosuppressive agents.

Treatment of a disease, according to the invention, refers to a biological effect that may present as a decrease in disease burden, disease incidence or disease severity. For example, in relation to cancer treatment, this may manifest as a reduction in tumour volume, a decrease in the number of tumour cells, a decrease in tumour cell proliferation, a decrease in the number of metastases, an increase in overall or progression-free survival, an increase in life expectancy, or amelioration of various physiological symptoms associated with the tumour.

The engineered cells of the invention may be administered by any appropriate route. Generally, the cells will be administered by intravenous infusion,

### Methods for treating cancer

In preferred embodiments of the invention, the cells of the invention are for use in treating cancer. The ability of pDCs to secrete immunomodulatory factors and alter immune-inhibiting tumour microenvironments are expected to be particularly useful for treating cancer. Also, cancer cells present specific antigens that can be recognised by the extracellular antigen recognition domain to provide targeted therapy at tumour sites, avoiding healthy cells.

In preferred embodiments, the cancer is a solid tumour. The cells of the invention may be particular effective at targeting and altering the microenvironment of solid tumours.

In certain embodiments, the cells of the invention are for use in treating acute lymphoblastic leukemia (ALL) (including non T cell ALL), acute myeloid leukemia, B cell prolymphocytic leukemia, B-cell acute lymphoid leukemia ("BALL"), blastic plasmacytoid dendritic cell neoplasm, Burkitf s lymphoma, chronic lymphocytic leukemia (CLL), chronic myelogenous leukemia (CML), chronic myeloid leukemia, chronic or acute leukemia, diffuse large B cell lymphoma (DLBCL), follicular lymphoma (FL), hairy cell leukemia, Hodgkin's Disease, malignant lymphoproliferative conditions, MALT lymphoma, mantle cell lymphoma, Marginal zone lymphoma, monoclonal gammapathy of undetermined significance (MGUS), multiple myeloma, myelodysplasia and myelodysplastic syndrome, non-Hodgkin's lymphoma (NHL), plasma cell proliferative disorder (including asymptomatic myeloma (smoldering multiple myeloma or indolent myeloma), plasmablastic lymphoma, plasmacytoid dendritic cell neoplasm, plasmacytomas (including plasma cell dyscrasia; solitary myeloma; solitary plasmacytoma; extramedullary plasmacytoma; and multiple plasmacytoma), POEMS syndrome (also known as Crow-Fukase syndrome; Takatsuki disease; and PEP syndrome), primary mediastinal large B cell lymphoma (PMBC), small cell- or a large cell-follicular lymphoma, splenic marginal zone lymphoma (SMZL), systemic amyloid light chain amyloidosis, T-cell acute lymphoid leukemia ("TALL"), T-cell lymphoma, transformed follicular lymphoma, or Waldenstrom macroglobulinemia, or a combination thereof. In some embodiments, the cancer is a myeloma. In one particular embodiment, the cancer is multiple myeloma. In some embodiments, the cancer is leukemia. In some embodiments, the cancer is acute myeloid leukemia. In some embodiments, the cancer is relapsed or refractory large B-cell lymphoma, diffuse large B-cell lymphoma (DLBCL) not otherwise specified, primary mediastinal large B-cell lymphoma, high grade B-cell lymphoma, or DLBCL arising from follicular lymphoma.

The extracellular antigen recognition domain in the exogenous construct is selected to bind an antigen expressed on the surface of the cancer to be treated. Exemplary extracellular antigen recognition domains are described above.

In some embodiments, the engineered cell of the invention is for use in methods that further comprise administering a chemotherapeutic. In certain embodiments, the chemotherapeutic selected is a lymphodepleting (preconditioning) chemotherapeutic, and is preferably administered before the cells of the invention. Such administration of a chemotherapeutic may improve survival of the transplanted cells.

In preferred embodiments, cell of the invention is for use in treating cancer and the cell is an engineered stem cell-derived plasmacytoid dendritic cell that expresses an exogenous chimeric antigen receptor or synNotch receptor comprising an extracellular antigen recognition domain that recognises a tumour associated antigen, a transmembrane domain, and an intracellular signalling domain that activates an anti-tumour immune response in the cell when the extracellular antigen recognition domain binds the target cell.

In the invention, engineered cells are administered to a subject already suffering from cancer, in an amount sufficient to cure, alleviate or partially arrest the cancer or one or more of its symptoms. Such therapeutic treatment may result in remission, stabilisation, reduction in metastasis or elimination of the cancer. An amount adequate to accomplish this is defined as "therapeutically effective amount". The subject may have been identified as suffering from cancer and being suitable for an adoptive cell transfer immunotherapy by any suitable means.

### Methods for treating autoimmune and inflammatory diseases

In preferred embodiments of the invention, the cells of the invention are for use in treating an autoimmune or inflammatory disease. The ability of pDCs to secrete immunomodulatory factors and alter inflammatory tissues or sites of autoimmune attack are expected to be particularly useful for treating autoimmune and inflammatory diseases. Also, autoimmune diseases are often triggered by specific autoantigens that are targeted by the patient's immune system and these autoantigens can be recognised by the extracellular antigen recognition domain to provide targeted therapy to sites of inflammation. Similarly, specific tissues that are inflamed, or that have been transplanted, can be targeted using the extracellular antigen recognition domain in the cells of the invention.

Targeting engineered pDCs to inflamed tissue and sites of inflammatory disease is expected to be effective because depletion of pDCs (in a murine asthma model) leads to T cell-mediated hyper-responsiveness, resulting in breakdown of tolerance (see Jan de Heer, 2004, J Exp Med, 200(1):89-98). pDCs are also important for long-term graft survival after allogeneic stem cell transplantation (see Peric et al. Biol Blood Marrow Transplant, 2015, 21(8), Goncalves et al. Biol Blood Marrow Transplant, 21(7), and Waller et al. J Clin Oncol, 2014, 32(22)). Also, high expression of PD-L1/CD86 in pDCs correlate with increased Tregs in liver transplant tolerance (see Tokita et al., Transplantation. 2008, 85(3):369-77). Also, adoptive transfer (pre-operative) of donor pDCs highly promotes heart transplant survival (see PMID: Björck et al. J Heart Lung Transplant, 2005, 24(8) and Abe et al., Am J Transplant, 2005, 5(8)).

In certain embodiments, the autoimmune disease is selected from the list consisting of: type 1 diabetes, thyroid autoimmune diseases (e.g. Hashimoto's and Graves'), Addison's adrenal insufficiency, oophoritis, orchitis, lymphocytic hypophysitis, autoimmune hypoparathyroidism, autoimmune hypoparathyroidism, Goodpasture's disease, autoimmune myocarditis, membranous nephropathy, autoimmune hepatitis, ulcerative colitis, Crohn's disease, multiple sclerosis, myasthenia gravis, neuromyelitis optica.

In certain embodiments, the inflammatory disease is selected from the list consisting of: cystic fibrosis, chronic inflammatory intestinal diseases like, for example, ulcerative colitis or Crohn's disease, vasculitis, in particular Kawasaki disease, chronic bronchitis, inflammatory arthritis diseases like, for example, psoriatic arthritis, osteoarthritis, rheumatoid arthritis, and systemic onset juvenile rheumatoid arthritis (SOJRA, Still's disease), graft-versus-host disease, asthma, psoriasis, systemic lupus erythematosus and allograft rejection.

In certain embodiments, the autoimmune or inflammatory disease is transplant rejection or graft-versus-host-disease (GVHD). Exemplary organ transplant to be treated according to the invention include: kidney, heart, lung, liver, intestine, pancreas and islet of Langerhans.

The extracellular antigen recognition domain in the exogenous construct is selected to target the autoantigen associated with the disease to be treated. Exemplary antigens to be targeted include (with the associated disease to be treated in parenthesis): GAD65 (type 1 diabetes), insulinoma associated protein 2 - IA-2 (type 1 diabetes), thyroid peroxidase - TPO (thyroid autoimmune diseases, Hashimoto's and Graves'), thyrotropin receptor - TSHR (thyroid autoimmune diseases, Hashimoto's and Graves'), adrenocorticotropic hormone receptor - ACTHR (Addison's adrenal insufficiency), 21-hydroxylase (Addison's adrenal insufficiency), 17-alpha-hydroxylase (oophoritis), P450 side-chain cleavage enzyme (oophoritis), sperm antigens (orchitis), pituitary cytosolic protein (lymphocytic hypophysitis), calcium sensing receptor - CaSR (autoimmune hypoparathyroidism), NACHT LRR and PYD domains-containing protein 5 - NALP5 (autoimmune hypoparathyroidism), alpha 3 chain of type IV collagen (Goodpasture's disease), myosin (Autoimmune myocarditis), Type-M phospholipase A2 receptor - PLA2R (membranous nephropathy), Cytochrome P450 1A2 (autoimmune hepatitis), tropomyosin isoform 5 - hTM5 (ulcerative colitis), major zymogen granule membrane glycoprotein 2 -GP2 (Crohn's disease), myelin basic protein - MBP (multiple sclerosis), nicotinic acetylcholine receptor - AChR (myasthenia gravis), aquaporin-4 - AQP4 (neuromyelitis optica).

When used to treat transplant rejection, the extracellular antigen recognition domain in the exogenous construct is selected to target the appropriate organ. Exemplary antigens to be targeted include (with the associated organ in parenthesis): GAD65 (pancreas or islet of Langerhans), insulinoma associated protein 2 - IA-2, (pancreas or islet of Langerhans), type-M phospholipase A2 receptor - PLA2R (kidney), myosin (heart), alpha 3 chain of type IV collagen (lung), cytochrome P450 1A2 (liver), major zymogen granule membrane glycoprotein 2 - GP2 (intestine), tropomyosin isoform 5 - hTM5 (intestine).

In preferred embodiments, the cell of the invention is for use in treating an autoimmune or inflammatory disease and the cell is an engineered stem cell-derived plasmacytoid dendritic cell that expresses an exogenous chimeric antigen receptor or synNotch receptor comprising an extracellular antigen recognition domain that recognises an autoantigen or a tissue specific antigen, a transmembrane domain, and an intracellular signalling domain that activates an anti-inflammatory immune response in the cell when the extracellular antigen recognition domain binds the target cell.

In the invention, engineered cells are administered to a subject already suffering from an autoimmune disease, in an amount sufficient to cure, alleviate or reduce the frequency of one or more symptoms. An amount adequate to accomplish this is defined as "therapeutically effective amount". The subject may have been identified as suffering from an autoimmune disease and being suitable for an adoptive cell transfer immunotherapy by any suitable means.

### Methods for generating cells of the invention

The engineered pDCs may be generated by any appropriate method. Exemplary methods for generating pDCs in significant amounts are provided in WO2018/206577. The invention also provide methods of generating engineered plasmacytoid dendritic cells.

In certain embodiments, the method for producing an engineered plasmacytoid dendritic cell (pDCs) comprises:
- providing hematopoietic stem progenitor cells (HSPCs)
- transforming said HSPCs with an exogenous construct comprising an extracellular antigen recognition domain that recognises an antigen on a target cell,
- incubating said HSPCs in one or more media, which media may typically comprise one or more cytokines, growth factors, interferons (IFNs) and/or aryl hydrocarbon receptor (AHR) antagonists (such as stemregenin-1), whereby said HSPCs are differentiated into precursor-pDCs and into pDCs.

In certain embodiments, the method for producing an engineered plasmacytoid dendritic cell (pDCs) comprises:
- providing hematopoietic stem progenitor cells (HSPCs),
- incubating said HSPCs in one or more media, which media may typically comprise one or more cytokines, growth factors, interferons (IFNs) and/or aryl hydrocarbon receptor (AHR) antagonists (such as stemregenin-1), whereby said HSPCs are differentiated into precursor-pDCs and into pDCs,
- transforming said pDCs with an exogenous construct comprising an extracellular antigen recognition domain that recognises an antigen on a target cell.

Accordingly, in certain embodiments, the method for producing an engineered plasmacytoid dendritic cell (pDCs) comprises:
- providing hematopoietic stem progenitor cells (HSPCs),
- incubating said HSPCs in one or more media, which media may typically comprise one or more cytokines, growth factors, interferons (IFNs) and/or aryl hydrocarbon receptor (AHR) antagonists (such as stemregenin-1), whereby said HSPCs are differentiated into precursor-pDCs and into pDCs, and
- transforming said HSPCs prior to differentiation, or transforming said pDCs subsequent to differentiation, with an exogenous construct comprising an extracellular antigen recognition domain that recognises an antigen on a target cell.

Transforming the cells can be achieved by any appropriate technique.

In some embodiments the exogenous construct is a viral construct. In some embodiments, the viral construct is an AAV construct, an adenoviral construct, a lentiviral construct, or a retroviral construct. The construct may comprise a reporter gene such as GFP, mCherry, truncated EGFR, or truncated tNGFR, or the extracellular domain of the CAR or synNotch may contain an epitope, to aid sorting of pDCs with the construct. In some embodiments, the viral construct is a lentiviral construct and transduction is performed using retronectin-coated plates or using lentiboost and protamine sulfate.

In preferred embodiments, CD34+ HSPC are transformed and then differentiated into pDCs.

In certain embodiments, the method for producing an engineered plasmacytoid dendritic cell (pDCs) comprises:
- providing hematopoietic stem progenitor cells (HSPCs)
- transforming said HSPCs with an exogenous construct comprising an extracellular antigen recognition domain that recognises an antigen on a target cell,
- incubating said HSPCs in a first medium comprising cytokines and growth factor whereby said HSPCs are differentiated into precursor-pDCs
- adding interferons (IFNs) to said first medium to obtain a second medium whereby said precursor- pDCs are differentiated into pDCs

In certain embodiments, the method for producing an engineered plasmacytoid dendritic cell (pDCs) comprises:
- providing hematopoietic stem progenitor cells (HSPCs)
- transforming said HSPCs with an exogenous construct comprising an extracellular antigen recognition domain that recognises an antigen on a target cell,
- incubating said HSPCs in a first medium comprising cytokines and growth factor whereby said HSPCs are differentiated into precursor-pDCs
- adding stem cell factor (SCF) and StemRegnin 1 (SR1) in a first medium to obtain high yield of pre-cursor pDCs
- providing a second medium comprising interferons (IFNs)
- adding interferons (IFNs) to said a second medium to said first medium comprising pre-cursor pDCs, whereby said precursor- pDCs are transformed into to obtain a high yield of fully activated and differentiated pDCs a second medium
whereby said precursor- pDCs are differentiated into pDCs

In preferred embodiments said second medium comprises IFN-γ and/or IFN-β. In another embodiment said second medium further comprises IL-3. Preferably, said second medium comprises IL-3, IFN-γ and IFN-β.

The precursor-pDCs may for example be incubated for at least 24 hours in said second medium. Preferably, said precursor-pDCs are incubated for 24 to 72 hours in said second medium.

In one embodiment said first medium comprises Flt3 ligand, thrombopoietin and/or interleukin-3. In another embodiment said first medium further comprises stem cell factor and StemRegenin 1. In another embodiment said first medium further comprises stem cell factor and UM 171. In another embodiment said first medium further comprises RPMI medium supplemented with fetal calf serum (FCS). In another embodiment said first medium comprises serum-free medium (SFEM). Preferably, said first medium comprises Flt3 ligand, thrombopoietin, SCF, interleukin-3 and StemRegenin 1.

The HSPCs may for example be incubated for 21 days in said first medium.

In one embodiment the method as described herein, further comprises a step of immunomagnetic negative selection to enrich for differentiated pDCs.

"Hematopoietic stem cells" (HSCs) as used herein are multipotent stem cells that are capable of giving rise to all blood cell types including myeloid lineages and lymphoid lineages. Myeloid lineages may for example include monocytes and macrophages, neutrophils, basophils, eosinophils, erythrocytes, megakaryocytes/platelets and dendritic cells, whereas lymphoid lineages may include T-cells, B-cells and NK-cells.

In a preferred embodiment HSCs are Hematopoietic stem and progenitor cells (HSPCs). HSCs or HSPCs are found in the bone marrow of humans, such as in the pelvis, femur, and sternum. They are also found in umbilical cord blood and in peripheral blood.

Stem and progenitor cells can be taken from the pelvis, at the iliac crest, using a needle and syringe. The cells can be removed as liquid for example to perform a smear to look at the cell morphology or they can be removed via a core biopsy for example to maintain the architecture or relationship of the cells to each other and to the bone.

The HSCs or HSPCs may also be harvested from peripheral blood. To harvest HSCs or HSPCs from the circulating peripheral blood, blood donors can be injected with a cytokine that induces cells to leave the bone marrow and circulate in the blood vessels. The cytokine may for example be selected from the group consisting of granulocyte-colony stimulating factor (G-CSF), GM-CSF granulocyte-macrophage colony-stimulating factor (GM-CSF) and cyclophosphamide. They are usually given as an injection into the fatty tissue under the skin every day for about 4-6 days.

The HSCs or HSPCs may also be harvested or purified from bone marrow. Stem cells are 10-100 times more concentrated in bone marrow than in peripheral blood. The hip (pelvic) bone contains the largest amount of active marrow in the body and large numbers of stem cells. Harvesting stem cells from the bone marrow is usually done in the operating room.

HSCs or HSPCs may also be purified from human umbilical cord blood (UCB). In this method, blood is collected from the umbilical cord shortly after a baby is born. The volume of stem cells collected per donation is quite small, so these cells are usually used for children or small adults.

The first medium is a differentiation medium, wherein HSCs are differentiated into precursor-pDCs. Thus, the first medium comprises differentiation factors.

Before differentiation of HSCs into precursor-pDCs, the HSCs may be cultured in a culture medium not comprising differentiation factors. The culture medium may be supplemented with conventional cell culture components such as serum, such as for example fetal calf serum, b-mercaptoethanol, antibiotics, such as penicillin and/or streptomycin, nutrients, and/or nonessential amino acids. Conventional cell culture components can also be substituted for conventional serum-free medium supplemented with conventional penicillin and/or streptomycin.

To initiate differentiation of HSCs into precursor-pDCs, differentiation factors, such as Flt3 ligand, thrombopoietin and/or at least one interleukin selected from interleukin-3, IFN-b and PGE2 are added to the medium. SCF and/or SR1 can also be used.

Thus, in a preferred embodiment said first medium comprises Flt3 ligand, thrombopoietin and/or at least one interleukin selected from interleukin-3, IFN-b and PGE2. More preferably, said first medium comprises Flt3 ligand, thrombopoietin and/or interleukin-3. In another preferred embodiment, the first medium comprises SCF and/or SR1

Appropriate culture media can be prepared by the skilled person, for example using the guidance in WO 2018/206577.

The HSPCs are incubated in the first medium under conditions that are typical for human cell cultures and well known to the skilled person. Typical conditions for incubation of cell cultures are for example a temperature of 37 °C, 95% humidity and 5% CO₂.

In one embodiment the HSPCs are incubated for at least 1 day, such as at least 2 days, at least 3 days, such as for example at least 4 days, such as at least 5 days, at least 6 days, such as for example at least 7 days, such as at least 8 days, at least 9 days, such as for example at least 10 days, such as at least 12 days, at least 14 days in said first medium. In a more preferred embodiment the culture is incubated for at least 16 days, such as at least 18 days, at least 20 days or such as for example at least 21 days in said first medium.

The HSCs may for example be incubated for 1 week, 2 weeks, 3 weeks or 4 weeks in said first medium. In a preferred embodiment said HSPCs are incubated for 21 days in said first medium.

In one embodiment the first medium is refreshed during the incubation period. The medium may for example be refreshed every second day, every third day or every fourth day during the incubation period. The first medium is preferably refreshed with medium containing one or more components of the first medium as described herein and above. Preferably the medium is refreshed with medium comprising the cytokines.

After incubation of HSPCs in the first medium, wherein HSCs are differentiated into precursor-pDCs, IFNs are added to the first medium thereby obtaining a second medium.

Alternatively, a second medium is provided, which comprises IFNs, such as IFN type I, IFN type II and/or IFN type III.

In one embodiment said second medium comprises IFN-α, IFN-γ and/or IFN-β.

In one preferred embodiment said second medium comprises IFN-γ and/or IFN-β. Preferably, said second medium comprises IFN-γ and IFN-β.

In another preferred embodiment said second medium comprises interleukin-3 (IL-3). In the embodiment, wherein the first medium comprises IL-3, IL-3 may be added to the medium again, for example together with the interferons. It is understood that the three components can be added in any order. In a particular preferred embodiment said second medium comprises IFN-γ, IFN-β and IL-3.

The precursor-pDCs are incubated in the second medium under conditions that are typical for human cell cultures and well known to the skilled person. Typical conditions for incubation of cell cultures are for example a temperature of 37 °C, 95% humidity and 5% CO₂.

In one embodiment said precursor-pDCs are incubated in said second medium for at least 1 hour, such as at least 5 hours, such as for example at least 10 hours, such as at least 15 hours or such as at least 20 hours in said second medium. In one preferred embodiment precursor-pDCs are incubated for at least 24 hours in said second medium.

In another embodiment said precursor-pDCs are incubated in said second medium for at least 1 day, at least two days, at least three days or at least 4 days.

### General

It is to be understood that different applications of the disclosed products and methods may be tailored to the specific needs in the art. It is also to be understood that the terminology used herein is for the purpose of describing particular embodiments of the invention only, and is not intended to be limiting.

In addition as used in this specification and the appended claims, the singular forms "a", "an", and "the" include plural referents unless the content clearly dictates otherwise. Thus, for example, reference to "a polypeptide" includes "polypeptides", and the like.

Unless specifically prohibited, the steps of a method disclosed herein may be performed in any appropriate order and the order in which the steps are listed should not be considered limiting.

### Example 1

Hematopoietic stem and progenitor cells HSPCs were electroporated with ribonucleoprotein (RNP) complexes consisting of Cas9 enzyme sgRNA directed against CCR5. Subsequently, cells were transduced with adeno-associated virus 6 (AAV6) carrying the CAR construct flanked by CCR5 homology arms for homology-directed repair displayed in Figure 7A. Successively, cells were differentiated to SC-pDCs. Figure 7B presents a representative FACS plot, showing expression of the anti-CD19 CAR construct in SC-pDCs (mock = cells that did not receive the AAV6 vector). Figure 7C presents a column diagram showing percentage of anti-CD19 CAR+ SC-pDCs for three donors.

This Example demonstrates that a conventional CAR can be expressed in pDCs, in particular pDCs differentiated from HSPCs transformed with the CAR construct.

### Example 2

Primed SC-pDCs, where 30% of the cells express the anti-CD19 CAR (RNP+donor), or SC-pDCs not expressing the construct (mock + donor), were stimulated for 20 hours with agonists directed against TLR7 (R837), TLR9 (CpGA) or remained unstimulated (UT).

Supernatants were subsequently harvested and bioactive type IIFN was evaluated using a commercially-available type I IFN bioassay (HEK-blue Type I IFN reporter bioassay).

The data in Figure 8 show that the type I IFN response (one of the hallmarks of pDC activation) is not affected in a population of SC-pDCs expressing an anti-CD19 CAR.

### Example 3

SC-pDCs, where 30% of the cells express an anti-CD19 CAR (RNP+donor), or SC-pDCs not expressing the construct (mock+donor), were primed for three days in medium supplemented with type I and II IFN (Figure 9A) or left non-primed (Figure 9B). Cells were subsequently co-cultured with the CD19+ target cell line (NALM-6) at an effector:target ratio of 1:1. Target cells were also seeded without effector cells (0:1) to exclude potential background. After 20 hours, supernatants were harvested and levels of the cytokines IFN-beta, IL-6, CXCL10 and TNF-alpha were quantified using a Mesoscale multiplex cytokine assay (MSD). The data are from one donor.

The data show that the population of SC-pDCs expressing an anti-CD19 CAR recognize the target cell line, and subsequently become activated to produce IFN-beta and pro-inflammatory factors (IL-6, CXCL10 and TNF-alpha).

### Example 4

In order to confirm that SC-pDCs can be transduced with SynNotch components and successfully express both receptor and response element, SynNotch SC-pDCs were generated, as shown schematically in Figure 10.

Hematopoietic stem and progenitor cells (HSPCs) derived from cord blood were thawed and pre-expanded at low density for 3 days. After 3-days of pre-expansion, 100,000 cells were co-transduced with lenti-viruses carrying a CD19-SynNotch receptor or a IL-12 SynNotch response element, as shown in Figure 11. Cells were transduced using retronectin-coated plates, or using lentiboost and protamine sulfate. 24-hours post transduction HSPCs were washed and resuspended in pDC differentiation medium, and pDC differentiation was initiated. After 16 days of culture, pDCs were isolated and specific co-culture setups were performed.

Figure 12 shows that the SC-pDCs successfully express both the SynNotch receptor and the SynNotch response element. Based on these data and the data in the other examples, it is expected that pDCs will also successfully express alternative exogenous constructs with extracellular antigen recognition domains, in particular CAR-T constructs and SynNotch receptors against alternative targets, and will also successfully express alternative response elements, in particular those with alternative inducible transgenes. This is because it is expected that any such constructs will be transduced into the HSPCs and expressed by the pDCs in substantially the same was as shown in the present example.

### Example 5

Activation of SynNotch SC-pDCs cells generated in accordance with the preceding example was analyzed in order to confirm that SynNotch components expressed by SC-pDCs are functional and that SynNotch SC-pDCs are effective for targeting and recognizing specific cells and activating a response, in particular an immune response, when recognizing specific cells.

Primed and non-primed synNotch SC-pDCs were co-cultured with different target cells and activation of the synNotch recepror was analysed by measuring IL-12 secretion using ELISA. The target:effector cell ratio was 5:1 (i.e. there were five times more target cells than effector cells). Supernatants were collected 24 hours later and activation of the synNotch response element was assessed by analysing levels of IL-12 by ELISA. The data are presented in Figure 13. The data are presented as OD values because the readings were outside of the standard curve.

Figure 13 demonstrates strong activation of the response element and secretion of IL-12 when the pDCs are contacted by with CD19+ target cells (NALM6 or REH6), but not when contacted with CD19-negative non-target cells (K562), or cultivated without cells (alone). These data confirm that the synNotch receptor and response element are functional in SC-pDCs and that SC-pDCs carrying the synNotch components can recognise target cells and activate an immune response upon binding the cells. Based on these data and the data in the other examples, it is expected that pDCs will also successfully recognize different cells when alternative extracellular antigen recognition domains or SynNotch receptors against alternative targets are used, because their expression in pDCs will be substantially the same as shown in the present example. Similarly, based on these data and the data in the other examples, it is expected that pDCs will also successfully activate alternative immune responses, if alternative constructs or inducible transgenes are used, because target cell binding and response induction will be substantially the same as shown in the present example. Finally, based on the robust and specific expression of IL-12 shown in the present example, and based on the data in the other examples, it is expected that pDCs will be effective for activating an immune response that is useful for treating disease.

### Example 6

The transduction and activation described in Examples 4 and 5 were repeated with HSPCs from another donor. After transduction of HSPCs, differentiation into pDCs, and 16 days of culture, as described in Example 4, SC-pDCs were isolated and primed before expression of synNotch receptor and response element was assessed on pDCs (lineage negative, CD11c negative, CD303+ cells), as shown in Figure 14. These data further confirm that pDCs are successfully transduced and can successfully express both the SynNotch receptor and the SynNotch response element.

Activation of the pDCs following contact with CD19+ target cells was analysed by measuring IL-12 secretion using ELISA. 100,000 primed pDCs were co-cultured with CD19+ NALM6 or REH6 target cells, or the non-target cells K562 (control), at a ratio of 1:1 and 1:3 effector: target. Supernatants were collected 20 hours after stimulation and levels of IL12 was analyzed using ELISA. The data are shown in Figure 15, which presents data for one donor and biological triplicates. These data show strong activation of the response element and secretion of IL-12 when the pDCs are contacted by with CD19+ target cells. These data also show that the activation is dose-dependent, with greater activation and secretion of IL-12 when more target cells are used. These data further confirm that pDCs can recognise target cells and activate an immune response upon binding the cells.

### Example 7

The transduction and activation described in Examples 4 and 5 were repeated with HSPCs from another donor. After transduction of HSPCs, differentiation into pDCs, and 16 days of culture, as described in Example 4, SC-pDCs were isolated and primed before expression of synNotch receptor and response element was assessed on SC-pDCs (lineage negative, CD11c negative, CD303+ cells), as shown in Figure 16. These data further confirm that pDCs are successfully transduced and can successfully express both the SynNotch receptor and the SynNotch response element.

Activation of SC-pDCs upon recognition of CD19+ target cells was then tested. 100,000 SynNotch SC-pDCs or non-transduced SC-pDCs (Mock) were seeded in a U-bottom 96-well plate, and subsequently overlayed with target cells at various effector:target ratios, e.g. 1: 1 (100,000 SC-pDCs: 100,000 target cells), 1:2 (100,000 SC-pDCs:200,000 target cells). As target cells, the CD19+ NALM6 or REH6 were used. As non-target cells, the CD19 negative K562 cell line was used. After 20 hours of co-culture, supernatants were collected and levels of IL-12 was assessed using an IL-12 ELISA. The data are presented in Figure 17 and they show a robust and specific response that is induced upon co-culture of pDCs with CD19+ cells and that is increased with higher effector:target cell ratios. These data further confirm that pDCs can recognise target cells and activate an immune response upon binding the cells.

Unless indicated otherwise, the methods used are standard biochemistry and molecular biology techniques.

## Claims

1. An engineered plasmacytoid dendritic cell for use in a method of treating a disease in a subject, wherein the method comprises administering the engineered cell to the subject,
wherein the cell expresses on its surface an exogenous construct comprising an extracellular antigen recognition domain that recognises an antigen on a target cell, and
wherein the cell activates an immune response when the extracellular antigen recognition domain binds the target cell.

2. The engineered plasmacytoid dendritic cell for use according to claim 1, wherein the disease is a cancer, an autoimmune disease, or an inflammatory disease.

3. An engineered plasmacytoid dendritic cell that expresses on its surface an exogenous construct comprising an extracellular antigen recognition domain that recognises an antigen on a target cell.

4. The engineered plasmacytoid dendritic cell for use according to claim 1 or claim 2, wherein the immune response comprises secretion of cytokines, such as pro-inflammatory or anti-inflammatory cytokines, or comprises recruitment or activation of host immune cells, such as Tregs or cytotoxic lymphocytes.

5. The engineered plasmacytoid dendritic cell for use or the engineered plasmacytoid dendritic cell according to any preceding claim, wherein the exogenous construct further comprises a transmembrane domain and an intracellular signalling domain that activates an immune response in the cell when the extracellular antigen recognition domain binds the target cell.

6. The engineered plasmacytoid dendritic cell for use or the engineered plasmacytoid dendritic cell according to claim 5, wherein the construct is a chimeric antigen receptor or a synthetic Notch receptor.

7. The engineered plasmacytoid dendritic cell for use or the engineered plasmacytoid dendritic cell according to claim 5 or claim 6, wherein the intracellular signalling domain comprises one or more TCR signalling components or comprises one or more transcription factors.

8. The engineered plasmacytoid dendritic cell for use or the engineered plasmacytoid dendritic cell according to any preceding claim, wherein the extracellular antigen recognition domain is a single chain antibody fragment (scFv) or a recombinant T cell receptor.

9. The engineered plasmacytoid dendritic cell for use or the engineered plasmacytoid dendritic cell according to any preceding claim, wherein the extracellular antigen recognition domain recognises a tumour associated antigen, a self-antigen associated with an autoimmune disease, or a tissue-specific antigen.

10. The engineered plasmacytoid dendritic cell for use or the engineered plasmacytoid dendritic cell according to any preceding claim, wherein the engineered plasmacytoid dendritic cell is a stem cell-derived plasmacytoid dendritic cell.

11. The engineered plasmacytoid dendritic cell for use or the engineered plasmacytoid dendritic cell according to any preceding claim, wherein the engineered plasmacytoid dendritic cell expresses TRAIL, CD123, CD303, CD304, CD4, HLA-DR, IFN type I, IFN type II, IFN type III, IRF7, TLR7 and/or TLR9.

12. A pharmaceutical composition comprising the engineered plasmacytoid dendritic cell of any of claims 3 or 5-11 and a pharmaceutically acceptable carrier.

13. The engineered plasmacytoid dendritic cell of any of any of claims 3 or 5-11, for use in a method of treating disease.

14. The engineered plasmacytoid dendritic cell of any of claims 3 or 5-11, for use in treating a cancer, an autoimmune disease or an inflammatory disease.

15. A method of generating a therapeutic composition comprising an engineered plasmacytoid dendritic cell comprising:
- providing hematopoietic stem progenitor cells (HSPCs),
- incubating said HSPCs in one or more media comprising cytokines, growth factor and/or interferons (IFNs), whereby said HSPCs are differentiated into precursor-pDCs and into pDCs incubating said HSPCs in one or more media, which media may comprise one or more cytokines, growth factors, interferons (IFNs) and/or aryl hydrocarbon receptor (AHR) antagonists (such as stemregenin-1), whereby said HSPCs are differentiated into precursor-pDCs and into pDCs, and
- transforming said HSPCs prior to differentiation, or transforming said pDCs subsequent to differentiation, with an exogenous construct comprising an extracellular antigen recognition domain that recognises an antigen on a target cell.

## Patentansprüche

1. Künstlich hergestellte plasmazytoide dendritische Zelle zur Verwendung in einem Verfahren zur Behandlung einer Krankheit bei einem Patienten, wobei das Verfahren die Verabreichung der künstlich hergestellten Zelle an den Patienten umfasst,
wobei die Zelle auf ihrer Oberfläche ein exogenes Konstrukt exprimiert, das eine extrazelluläre Antigenerfassungsdomäne umfasst, die ein Antigen auf einer Zielzelle erkennt, und
wobei die Zelle eine Immunantwort auslöst, wenn die extrazelluläre Antigenerfassungsdomäne die Zielzelle bindet.

2. Künstlich hergestellte plasmazytoide dendritische Zelle zur Verwendung nach Anspruch 1, wobei die Krankheit ein Krebs, eine Autoimmunerkrankung oder eine entzündliche Erkrankung ist.

3. Künstlich hergestellte plasmazytoide dendritische Zelle, die auf ihrer Oberfläche ein exogenes Konstrukt exprimiert, das eine extrazelluläre Antigenerfassungsdomäne umfasst, die ein Antigen auf einer Zielzelle erkennt.

4. Künstlich hergestellte plasmazytoide dendritische Zelle zur Verwendung nach Anspruch 1 oder Anspruch 2, wobei die Immunantwort die Sekretion von Zytokinen, wie z.B. pro- oder antiinflammatorischen Zytokinen, umfasst oder die Rekrutierung oder Aktivierung von Wirtsimmunzellen, wie z.B. Tregs oder zytotoxischen Lymphozyten, umfasst.

5. Künstlich hergestellte plasmazytoide dendritische Zelle zur Verwendung oder künstlich hergestellte plasmazytoide dendritische Zelle nach einem der vorhergehenden Ansprüche, wobei das exogene Konstrukt ferner eine Transmembrandomäne und eine intrazelluläre Signaldomäne umfasst, die eine Immunantwort in der Zelle aktiviert, wenn die extrazelluläre Antigenerfassungsdomäne an die Zielzelle bindet.

6. Künstlich hergestellte plasmazytoide dendritische Zelle zur Verwendung oder künstlich hergestellte plasmazytoide dendritische Zelle nach Anspruch 5, wobei das Konstrukt ein chimärer Antigenrezeptor oder ein synthetischer Notch-Rezeptor ist.

7. Künstlich hergestellte plasmazytoide dendritische Zelle zur Verwendung oder künstlich hergestellte plasmazytoide dendritische Zelle nach Anspruch 5 oder Anspruch 6, wobei die intrazelluläre Signaldomäne eine oder mehrere TCR-Signalkomponenten umfasst oder einen oder mehrere Transkriptionsfaktoren umfasst.

8. Künstlich hergestellte plasmazytoide dendritische Zelle zur Verwendung oder künstlich hergestellte plasmazytoide dendritische Zelle nach einem der vorhergehenden Ansprüche, wobei die extrazelluläre Antigenerfassungsdomäne ein einkettiges Antikörperfragment (scFv) oder ein rekombinanter T-Zell-Rezeptor ist.

9. Künstlich hergestellte plasmazytoide dendritische Zelle zur Verwendung oder künstlich hergestellte plasmazytoide dendritische Zelle nach einem der vorhergehenden Ansprüche, wobei die extrazelluläre Antigenerfassungsdomäne ein tumorassoziiertes Antigen, ein mit einer Autoimmunerkrankung assoziiertes Selbstantigen oder ein gewebespezifisches Antigen erkennt.

10. Künstlich hergestellte plasmazytoide dendritische Zelle zur Verwendung oder künstlich hergestellte plasmazytoide dendritische Zelle nach einem der vorhergehenden Ansprüche, wobei die künstlich hergestellte plasmazytoide dendritische Zelle eine von Stammzellen abgeleitete plasmazytoide dendritische Zelle ist.

11. Künstlich hergestellte plasmazytoide dendritische Zelle zur Verwendung oder künstlich hergestellte plasmazytoide dendritische Zelle nach einem der vorhergehenden Ansprüche, wobei die manipulierte plasmazytoide dendritische Zelle TRAIL, CD123, CD303, CD304, CD4, HLA-DR, IFN Typ I, IFN Typ II, IFN Typ III, IRF7, TLR7 und/oder TLR9 exprimiert.

12. Pharmazeutische Zusammensetzung, die die manipulierte plasmazytoide dendritische Zelle nach einem der Ansprüche 3 oder 5 - 11 und einen pharmazeutisch annehmbaren Träger umfasst.

13. Künstlich hergestellte plasmazytoide dendritische Zelle nach einem der Ansprüche 3 oder 5 - 11 zur Verwendung in einem Verfahren zur Behandlung einer Krankheit.

14. Künstlich hergestellte plasmazytoide dendritische Zelle nach einem der Ansprüche 3 oder 5 - 11 zur Verwendung bei der Behandlung eines Krebses, einer Autoimmunerkrankung oder einer entzündlichen Erkrankung.

15. Verfahren zur Herstellung einer therapeutischen Zusammensetzung, die eine manipulierte plasmazytoide dendritische Zelle umfasst, umfassend:
- Bereitstellen von hämatopoetischen Stammvorläuferzellen (HSPCs),
- Inkubieren der HSPCs in einem oder mehreren Medien, die Zytokine, Wachstumsfaktoren und/oder Interferone (IFNs) umfassen, wodurch die HSPCs in Vorläufer-pDCs und in pDCs differenziert werden, Inkubieren der HSPCs in einem oder mehreren Medien, wobei die Medien ein oder mehrere Zytokine, Wachstumsfaktoren, Interferone (IFNs) und/oder Aryl-Kohlenwasserstoff-Rezeptor (AHR)-Antagonisten (wie z.B. Stammregenin-1) umfassen können, wodurch die HSPCs in Vorläufer-pDCs und in pDCs differenziert werden, und
- Transformieren der HSPCs vor der Differenzierung, oder Transformieren der pDCs nach der Differenzierung mit einem exogenen Konstrukt, das eine extrazelluläre Antigenerfassungsdomäne umfasst, die ein Antigen auf einer Zielzelle erkennt.

## Revendications

1. Cellule dendritique plasmacytoïde modifiée à utiliser dans un procédé de traitement d'une maladie chez un sujet, le procédé comprenant l'administration de la cellule modifiée au sujet, dans laquelle la cellule exprime sur sa surface une construction exogène comprenant un domaine de reconnaissance d'antigène extracellulaire qui reconnaît un antigène sur une cellule cible, et dans laquelle la cellule exprime une réponse immunitaire lorsque le domaine de reconnaissance d'antigène extracellulaire se lie à la cellule cible.

2. Cellule dendritique plasmacytoïde modifiée à utiliser selon la revendication 1, dans laquelle la maladie est un cancer, une maladie auto-immune ou une maladie inflammatoire.

3. Cellule dendritique plasmacytoïde modifiée qui exprime à sa surface une construction exogène comprenant un domaine de reconnaissance d'antigène extracellulaire qui reconnaît un antigène sur une cellule cible.

4. Cellule dendritique plasmacytoïde modifiée à utiliser selon la revendication 1 ou la revendication 2, dans laquelle la réponse immunitaire comprend la sécrétion de cytokines, telles que des cytokines pro-inflammatoires ou anti-inflammatoires, ou comprend le recrutement ou l'activation de cellules immunitaires de l'hôte, telles que des Tregs ou des lymphocytes cytotoxiques.

5. Cellule dendritique plasmacytoïde modifiée à utiliser ou la cellule dendritique plasmacytoïde modifiée selon l'une quelconque des revendications précédentes, dans laquelle la construction exogène comprend en outre un domaine transmembranaire et un domaine de signalisation intracellulaire qui active une réponse immunitaire dans la cellule lorsque le domaine de reconnaissance d'antigène extracellulaire se lie à la cellule cible.

6. Cellule dendritique plasmacytoïde modifiée à utiliser ou la cellule dendritique plasmacytoïde modifiée selon la revendication 5, dans laquelle la construction est un récepteur d'antigène chimérique ou un récepteur Notch synthétique.

7. Cellule dendritique plasmacytoïde modifiée à utiliser ou la cellule dendritique plasmacytoïde modifiée selon la revendication 5 ou la revendication 6, dans laquelle le domaine de signalisation intracellulaire comprend un ou plusieurs composants de signalisation TCR ou comprend un ou plusieurs facteurs de transcription.

8. Cellule dendritique plasmacytoïde modifiée à utiliser ou la cellule dendritique plasmacytoïde modifiée selon l'une quelconque des revendications précédentes, dans laquelle le domaine de reconnaissance d'antigène extracellulaire est un fragment d'anticorps à chaîne unique (scFv) ou un récepteur de lymphocytes T recombinant.

9. Cellule dendritique plasmacytoïde modifiée à utiliser ou la cellule dendritique plasmacytoïde modifiée selon l'une quelconque des revendications précédentes, dans laquelle le domaine de reconnaissance d'antigène extracellulaire reconnaît un antigène associé à une tumeur, un auto-antigène associé à une maladie auto-immune ou un antigène spécifique d'un tissu.

10. Cellule dendritique plasmacytoïde modifiée à utiliser ou la cellule dendritique plasmacytoïde modifiée selon l'une quelconque des revendications précédentes, dans laquelle la cellule dendritique plasmacytoïde modifiée est une cellule dendritique plasmacytoïde dérivée de cellules souches.

11. Cellule dendritique plasmacytoïde modifiée à utiliser ou la cellule dendritique plasmacytoïde modifiée selon l'une quelconque des revendications précédentes, dans laquelle la cellule dendritique plasmacytoïde modifiée exprime TRAIL, CD123, CD303, CD304, CD4, HLA-DR, IFN type I, IFN type II, IFN type III, IRF7, TLR7 et/ou TLR9.

12. Composition pharmaceutique comprenant la cellule dendritique plasmacytoïde modifiée selon l'une quelconque des revendications 3 ou 5 à 11 et un support pharmaceutiquement acceptable.

13. Cellule dendritique plasmacytoïde modifiée selon l'une quelconque des revendications 3 ou 5 à 11, à utiliser dans un procédé de traitement de maladies.

14. Cellule dendritique plasmacytoïde modifiée selon l'une quelconque des revendications 3 ou 5 à 11, à utiliser dans le traitement d'un cancer, d'une maladie auto-immune ou d'une maladie inflammatoire.

15. Procédé de génération d'une composition thérapeutique comprenant une cellule dendritique plasmacytoïde modifiée comprenant :
- la fourniture de cellules souches hématopoïétiques progénitrices (HSPC),
- l'incubation desdites HSPC dans un ou plusieurs milieux comprenant des cytokines, un facteur de croissance et/ou des interférons (IFN), moyennant quoi lesdites HSPC sont différenciées en précurseurs pDC et en pDC incubant lesdites HSPC dans un ou plusieurs milieux, lesquels milieux peuvent comprendre une ou plusieurs cytokines, des facteurs de croissance, des interférons (IFN) et/ou des antagonistes des récepteurs des hydrocarbures aryliques (AHR) (tels que la stemrégénine-1), moyennant quoi lesdites HSPC sont différenciées en précurseurs- pDC et en pDC, et
- la transformation desdites HSPC avant la différenciation, ou la transformation desdites pDC après la différenciation, avec une construction exogène comprenant un domaine de reconnaissance d'antigène extracellulaire qui reconnaît un antigène sur une cellule cible.
